(19) Europäisches Patentamt
European Patent Office
Office européen des brevets

(11) **EP 4 764 497 A1**

(12) **EUROPEAN PATENT APPLICATION**

(43) Date of publication:
**24.06.2026 Bulletin 2026/26**

(21) Application number: **24307240.2**

(22) Date of filing: **20.12.2024**

(51) International Patent Classification (IPC):
**G01N 33/569** (2006.01) **G01N 27/447** (2006.01)

(52) Cooperative Patent Classification (CPC):
**G01N 33/56983;** G01N 27/44726; G01N 2333/15

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Geneuro SA**
**1228 Plan-Les-Ouates (CH)**
• **Institut National de la Santé et de la Recherche Médicale**
**75013 Paris (FR)**
• **Université Toulouse III - Paul Sabatier**
**31400 Toulouse (FR)**

(72) Inventors:
• **PERRON, Hervé**
**69290 SAINT GENIS LES OLLIÈRES (FR)**

• **CHARVET, Benjamin**
**69009 LYON (FR)**
• **MEDINA, Julie**
**69210 LENTILLY (FR)**
• **LUCAS, Alexandre**
**31700 CORNEBARRIEU (FR)**
• **FRIED, Steven**
**31400 TOULOUSE (FR)**

(74) Representative: **Plasseraud IP**
**104 Rue de Richelieu**
**CS92104**
**75080 Paris Cedex 02 (FR)**

Remarks:
The complete document including Reference Table(s) and the Sequence Listing(s) can be downloaded from the EPO website

(54) **HERV ENV DETECTION TEST**

(57) The present application deals with the quantitative detection of HERV ENV in a sample such as serum, more particularly the detection of HERV-W ENV or HERV-K ENV.

EP 4 764 497 A1

**Description**

**FIELD OF THE INVENTION**

The present application deals with the quantitative detection of HERV ENV in a sample such as serum, more particularly the detection of HERV-W ENV or HERV-K ENV.

**BACKGROUND OF THE INVENTION**

The envelope protein of HERV retroviruses (HERV ENV), such as HERV-W ENV or HERV-K ENV, can be expressed in some individuals due to environmental influence. Sometimes, its level of expression is such that it may be responsible for triggering certain pathologies, such as multiple sclerosis or amyotrophic lateral sclerosis. In its circulating form in biological fluids, HERV ENV can be found in oligomeric form, such as HERV-W ENV, which is present in hexameric form.

Until now, tests for detecting HERV ENV in biological fluids, such as HERV-W ENV or HERV-K ENV in serum, did not allow quantification of the protein, and only detected the presence or absence of the protein in a sample ("detection-only test"). A primary anti-HERV ENV antibody and a secondary antibody coupled to a marker were used to generate a sufficient signal. Due to background noise in these conditions, signal processing was necessary to obtain the test result.

It was therefore mandatory to obtain a reference panel of "healthy donors" to determine the non-specific background of the corresponding body fluid or sample, and a threshold to calculate a signal-to-noise ratio (S/N), for each series of analyses and for each site where the test was to be carried out (WO2019/201908).

From the panel of healthy controls, the mean and standard deviation of the non-specific background was calculated at the level of the molecular weight of the specific HERV-ENV peak. A "threshold" value (N=mean+2 standard deviations) for these controls, below which any signal was considered negative, was then determined, as possibly due to background noise linked to the method and the properties of normal serum. These constraints were incompatible with industrial development and marketing for routine diagnosis.

Furthermore, as there was no method for in vitro production of oligomeric recombinant HERV ENV proteins, a standard curve of native antigen could not be produced. There was therefore no possibility of making a "fully quantitative assay" that could be marketed with reagents in a kit, which would be self-sufficient in including the oligomerized recombinant protein, without the need to recruit healthy controls each time.

There was therefore a need to develop a quantitative detection test that was fast, marketable and easy to implement.

The inventors have discovered that the use of the directly labeled humanized primary antibody enabled background suppression. Thanks to the present diagnostic test, there is no need for S/N calculation anymore, and no need for a reference panel of "healthy donors" anymore.

Moreover, this new diagnostic test also enables the quantification of low concentrations, which had previously been impossible. Because of this background noise, previous tests were unable to quantify below the "N" (noise) threshold, which de facto became the "lower limit of quantification-LLOQ", i.e. the sensitivity criterion for the test and its method. This translated into the possibility of identifying weaker positives that were previously classified as "negative" ($S/N \leq 1$). In the novel conditions provided by the present invention with or without a standard of recombinant HERV ENV protein, i.e. with the sole use of this directly-labeled humanized primary antibody, such false negative samples of previously misclassified patients can be identified as truly positive ones. Indeed, detection of these patients falsely classified as negative with previous tests shows that these were not good tests, generating errors with serious consequences by giving false negative diagnostic results.

The diagnostic test developed by the inventors is much more sensitive and accurate than tests of the prior art, and thus avoids false negative results.

In addition, the inventors have succeeded for the first time in obtaining oligomers in vitro from monomers of recombinant HERV ENV proteins produced in E. coli. This allows, for the first time, the elaboration of a standard curve of purified recombinant antigen equivalent to the native antigen isolated in pathologies. This is a tool that has been lacking and is mandatory for a fully quantitative diagnostic test to be disseminated in numerous sites with a universal reference standard. In addition, it provides a test that can be industrially developed and marketed as a kit for routine diagnosis.

They also discovered a procedure for preparing the serum sample in one step for the diagnostic test, which allows a test-ready sample to be obtained in just a few hours, as opposed to two days in the past with a 3-step preparation (see Example 8.1).

**SUMMARY OF THE INVENTION**

The present invention relates to a novel test allowing an effective detection and quantification of envelope proteins from Human Endogenous Retroviruses (HERVs) families and that of other retroviruses, with optimized sensitivity, specificity and time-to result conditions suitable for routine diagnostic use in medical settings. This new test uses a novel and unique combination of converging knowledges acquired from (i) the discovery the physico-chemical properties of HERV envelopes proteins, (ii) the unexpected conditions of their one-step isolation from body fluids or tissues in various disease or physiological conditions under the form of oligomers or monomers, (iii) the use of an immunoassay platform allowing to separate molecules according to their molecular weight followed by an immunodetection visualized under the form of an electrophoregram, (iii) the unexpected finding of unusual conditions of use of a specific buffer that made it possible to obtain oligomerization of E. coli-produced and purified recombinant monomers of HERV envelope proteins, (iv) the feasibility of a quantification standard curve using such conditions generating oligomers from recombinant proteins with the same molecular weight as the retroviral envelope proteins extracted from body fluids or tissues, (v) the unexpected and major improvement of the immunodetection step using a humanized specific IgG4 antibody directly labelled with biotin followed by revelation with a peroxidase enzyme bound to streptavidin that eliminated the usual background noise yielding non-specific signal with primary and/or secondary mouse antibodies and thereby making it impossible to quantify the specific signal below a threshold corresponding to the maximal statistical level of this background noise on various body fluid or tissue extracts, (vi) the use of this immunodetection humanized antibody in combination with a technique allowing a physico-chemical capture (UV-induced aminoacid covalent bounds with a gel used in capillary tubes) of the viral envelope proteins, but not an immunological antigen capture as in sandwich ELISA techniques, (vii) the use of the oligomerized viral envelope recombinant proteins providing a dose response calibration curve to obtain an accurate and bioequivalent quantification of signals obtained from the same envelope antigens extracted with a new protocol from body fluids or tissue samples and, (viii) the reproducible and standardized inter-assay and multi-sites results on such samples analyzed with all these combined existing or discovered parameters, thereby making it a novel and unique test for the highly specific and sensitive routine-adapted diagnostic test quantifying HERV andretroviral envelope glycoproteins expressed under various conditions in human or animal tissues. This novel test is also making the detection and quantification of HERV, retroviral or viral envelope proteins from body fluids and tissues suitable as diagnostic tests for precision or individualized medicine indications as well as for therapeutic monitoring of treatments or drugs targeting HERV envelope proteins, as well as retroviral or viral expression that could use viral envelope proteins as biomarkers for the same purposes.

## DEFINITIONS

As used herein, the terms **"human endogenous retrovirus"** and **"HERV",** refer to the human endogenous retroviruses, that comprise for example viruses and chromosomal copies belonging to types W and K endogenous retrovirus families, usually named "HERV-W and HERV-K".

In the context of the present disclosure, the expressions **"HERV Env"** or **"HERV ENV"** both refer to the same type of protein as used herein, and both mean either envelope protein of HERV or human genome sequence encoding the envelope protein of HERV.

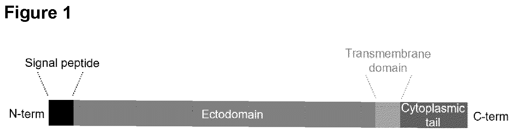

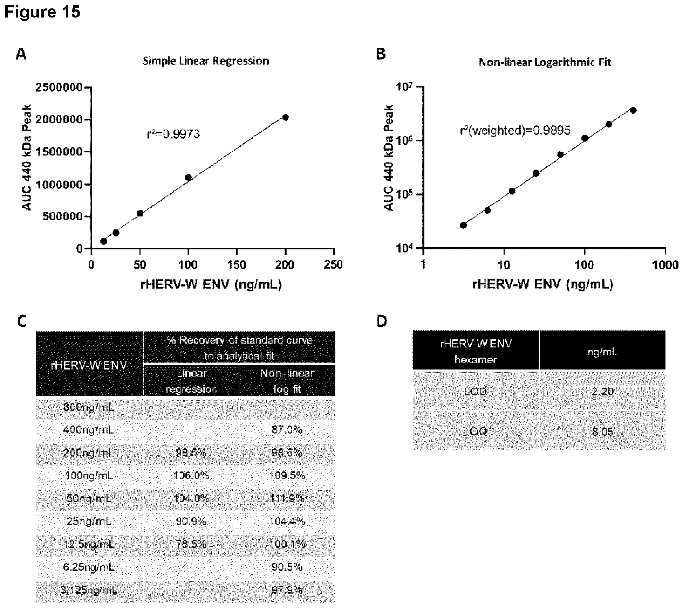

The classical HERV-ENV protein structure is illustrated in Figure 1. The envelope proteins (ENV) encoded by HERV-*ENV* genes present a global organization, starting from the N-term end towards the C-term end, composed of (i) a signal peptide allowing the targeting of the newly synthesized protein towards the Golgi apparatus and then its export towards the cell membrane. The signal peptide is cleaved thereafter, (ii) an ectodomain, here called SU, followed by (iii) a transmembrane domain and (iv) an intracytoplasmic domain (or "tail"). The association of the transmembrane and intracytoplasmic domains is here denominated as the TM part of the protein. A complete and mature ENV protein is therefore a protein composed of the SU-TM parts. In replication-competent retroviruses or in HERV envelope proteins that retained this property with corresponding functional sequence, a cleavage site for the furin enzyme is present upstream of the membrane-spanning amino-acids within the C-term part of the ectodomain (extracellular part at the membrane surface, here called SU, *stricto sensu*). In other denominations, the SU domain ends at this furin cleavage site and the TM domain encompasses the amino-acids of the ectodomain downstream of this cleavage site, the membrane spanning ones, and the intracytoplasmic tail. This representation shows that HERV envelope proteins may be expressed independently from other retroviral genes and can be produced, exposed at the cell surface and secreted extracellularly as complete and uncleaved SU-TM proteins, once the signal peptide has normally been removed. This is, e.g., the case for the HERV-W ENV protein as characterized from pathogenic expression in certain human diseases.

**"HERV-W"** is a family of human endogenous retroviruses that was unravelled in human genome from the initial discovery of "Multiple Sclerosis associated Retrovirus", MSRV, a human retrovirus first isolated from patients with multiple sclerosis. Therefore, when studies mention "LM7" (first isolate described from MS), "MS-retrovirus", "MSRV", "Syncytin", "HERV-W 7q", "ERVW-E1", "ERVW-E2", "HERV-W copies from X chromosome" or "HERV-W", they all designate elements from the HERV-W family.

As used herein, the term **"MSRV"** refers to a specific endogenous retrovirus which is a member of the HERV-W family. In the context of the present disclosure, the expressions **"HERV-W"** and **"MSRV"** both designate HERV-W elements. Specifically, the expressions **"HERV-W Env"** and **"MSRV-Env"** both refer to the same envelope proteins.

Typically, and whenever observed, few variations in amino acid sequence do not prevent the binding of specific anti-Env antibodies on the same epitope region for therapeutic or diagnostic use.

**"HERV-K"** is a family of endogenous retroviruses that were integrated into the human genome at least 30 million years ago and appears to still be active (Belshaw et al., 2004 PNAS). When studies mention **"HML-2", "HERV-K(HML-2)"**, they all designate HERV-K elements.

As used herein or in the literature, the terms **"HML-2", "HML-2 ENV", "HERV-K ENV"** or **"HERV-K113", "HERV-K113 ENV", "HERV-K6", "HERV-K18"** (including variants **"K18.1"** and **"K18.2"**), **"HERV-K18 ENV"** and all related terms refer to the same type of protein as used herein, meaning an envelope protein of HERV-K family or the genome sequence encoding the envelope protein of HERV-K family (Gröger et al., 2020 Int J Mol Sci).

As used herein, the term "**detecting**" or "**detection**", means identifying, or not, the presence of the protein of interest in a sample. The terms "**quantitative detection**" or "**quantification**" both mean that the initial detection goes beyond the simple conclusion on the absence or the presence of the protein of interest in the sample, it means that when the protein of interest is present in the sample, it is possible to quantify its concentration in the sample. A "**quantitative detection method**" means that the quantification obtained with a test using this method makes it possible to quantify low to high concentrations and provides values than can be accurately compared between samples of different samples of different individuals, or from the same individual at different timepoints.

As used herein, the term "**antibody**" refers to immunoglobulin molecules and immunologically active portions of immunoglobulin molecules, i.e., molecules that contain an antigen binding site that specifically binds an antigen. As such, the term "antibody" should be understood broadly and encompasses not only immunoglobulin molecules, but also antibody fragments, as well as derivatives of antibodies.

In natural antibodies, two heavy chains (HC) are linked to each other by disulfide bonds and each heavy chain is linked to a light chain (LC) by a disulfide bond. There are two types of light chain, lambda (I) and kappa (k). There are five main heavy chain classes (or isotypes) which determine the functional activity of an antibody molecule: IgM, IgD, IgG, IgA and IgE. Each chain contains distinct sequence domains.

Typically, the light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine the binding site specific to the antigenic epitope. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody binding site and the antigenic epitope. Antibody binding sites are made up of residues that are primarily from the hypervariable or complementarity determining regions (CDRs). Complementarity Determining Regions or CDRs refer to amino acid sequences which together define the binding affinity and specificity of the natural Fv region of a native immunoglobulin binding site. The light and heavy chains of an immunoglobulin each have three CDRs, designated CDR-L1, CDR-L2, CDR-L3 and CDR-H1 , CDR-H2, CDR-H3, respectively. An antigen-binding site, therefore, includes six CDRs, comprising the CDR set from each of a heavy and a light chain V region. Framework Regions (FRs) refer to amino acid sequences interposed between CDRs.

As used herein, the expression "fragment of antibody" refers to a portion of such an antibody that mimics the hypervariable region, such as a CDR (CDR-L1, CDR-L2, CDR-L3, CDR-H1, CDR-H2, CDR-H3). The fragments of antibody retain the binding affinity and specificity of said antibody. Such fragments are functional equivalents of said antibody and they bind substantially to the same epitope as said antibody. Examples of fragments of antibody include but are not limited to heavy chain, light chain, VL, VH, Fv, Fab, Fab', F(ab)2, and F(ab')2.

The term "Fab" denotes an antibody fragment having a molecular weight of about 50,000 and antigen binding activity, in which about a half of the N-terminal side of H chain and the entire L chain, among fragments obtained by treating IgG with a protease, papain, are bound together through a disulfide bond.

As used herein, the term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin.

As used herein, the term "Fab' " refers to an antibody fragment having a molecular weight of about 50,000 and antigen binding activity, which is obtained by cutting a disulfide bond of the hinge region of the F(ab')2.

The expressions "A single chain Fv" or "scFv"" refer to a polypeptide which is a covalently linked VH::VL heterodimer, and usually expressed from a gene fusion including VH and VL encoding genes linked by a peptide-encoding linker. "dsFv" is a VH::VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)2.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

As used herein, the expression "derivative of antibody" refers to a fragment of the antibody, preferably including the six CDRs of said antibody, fused to at least one sequence different from the natural sequence (e.g. a linker sequence of another species...), which conserves a comparable binding affinity and specificity to HERV-W ENV or HERV-K ENV. Such derivatives are functional equivalents of said antibody and they bind at substantially the same epitope as said antibody. Examples of derivatives of antibody include, but are not limited to scFv, (scFv)2 and diabodies.

According to the disclosure, the term "humanized antibody" refers to an antibody having variable region framework and constant regions from a human antibody but retains the CDRs of an antibody from any species, preferably mouse.

## DETAILED DESCRIPTION

According to a first aspect, the present disclosure relates to a quantitative detection method of HERV ENV in a sample to be analyzed, with a capillary immunoelectrophoresis device and based on the use of a single and biotinylated antibody specific for HERV ENV detection with a marker conjugated to streptavidin.

Thus, the present disclosure relates to a quantitative detection method of HERV ENV in a sample to be analyzed, with a capillary immunoelectrophoresis device and the use of an anti-HERV ENV biotinylated antibody and a marker conjugated to streptavidin, as compared to a standard of recombinant HERV ENV protein.

A biotinylated antibody is an antibody linked to biotin. The binding of biotin to streptavidin is one of the strongest non-covalent interactions known in nature, with a dissociation constant (Kd) on the order of $10^{-14}$ mol/L. The present disclosure also covers any other pairs of proteins with a dissociation constant (Kd) on the order of $10^{-14}$ mol/L, such as avidin and biotin.

In particular, it relates to the detection of HERV-W ENV. In this embodiment the biotinylated anti-HERV ENV antibody biotinylated antibody is an anti-HERV-W ENV biotinylated antibody.

For example, it is an antibody which comprises each of the complementary-determining regions (CDRs) set forth in SEQ ID NO: 1, SEQ ID NO: 2, SEQ ID NO: 3 SEQ ID NO: 4, SEQ ID NO: 5 and SEQ ID NO: 6.

More particularly, said antibody comprises:

- a light chain comprising a light chain variable region (VL) which comprises three complementarity determining regions: CDR-L1 as set forth in SEQ ID NO:1, CDR-L2 as set forth in SEQ ID NO:2 and CDR-L3 as set forth in SEQ ID NO:3, and

- a heavy chain comprising a heavy chain variable region (VH) which comprises three complementarity determining regions: CDR-H1 as set forth in SEQ ID NO:4, CDR-H2 as set forth in SEQ ID NO:5 and CDR-H3 as set forth in SEQ ID NO:6.

In particular, the anti-HERV-W ENV antibody is a humanized antibody.

In particular, the anti-HERV-W ENV antibody is an IgG, such as IgG1 or IgG4.

More particularly, said antibody comprises:

- a light chain comprising a light chain variable region (VL) as depicted in SEQ ID NO:7, and

- a heavy chain comprising a heavy chain variable region (VH) as depicted in SEQ ID NO:8.

For example, the anti-HERV-W ENV antibody is the humanized anti-HERV-W ENV antibody called Temelimab.

In particular, it relates to the detection of HERV-K ENV. In this embodiment the anti-HERV ENV biotinylated antibody is an anti-HERV-K ENV biotinylated antibody.

For example, it is an antibody which comprises each of the complementary-determining regions (CDRs) set forth in SEQ ID NO: 9, SEQ ID NO: 10, SEQ ID NO: 11 SEQ ID NO: 12, SEQ ID NO: 13 and SEQ ID NO: 14.

More particularly, said antibody comprises:

- a light chain comprising a light chain variable region (VL) which comprises three complementarity determining regions: CDR-L1 as set forth in SEQ ID NO:9, CDR-L2 as set forth in SEQ ID NO:10 and CDR-L3 as set forth in SEQ ID NO:11, and

- a heavy chain comprising a heavy chain variable region (VH) which comprises three complementarity determining regions: CDR-H1 as set forth in SEQ ID NO:12, CDR-H2 as set forth in SEQ ID NO:13 and CDR-H3 as set forth in SEQ ID NO:14.

In particular, the anti-HERV-K ENV antibody is a humanized antibody.

In particular, the anti-HERV-K ENV antibody is an IgG, such as IgG1 ou IgG4.

Typically, the sample to be analyzed is serum or plasma. In particular, the sample serum.

The marker may be an enzymatic, fluorescent, chemiluminescent, radioactive or dye markers. It can be also any other marker known in the art which allows a revelation with a light or color emission. Typically, the marker is a peroxidase with a light-generating substrate, such as a chemiluminescent substrate. For example, the horseradish peroxidase (HRP).

In particular, the standard of recombinant HERV ENV protein, also called reference, used to quantify HERV ENV in the sample to be analyzed, is a calibration curve of recombinant HERV ENV protein.

In the present disclosure, calibration curve may be also called standard curve. Both expressions have the same meaning and are interchangeable. Thus, in particular the standard of recombinant HERV ENV protein is a calibration curve with recombinant HERV ENV protein, preferably with oligomeric recombinant HERV ENV protein.

The calibration curve is obtained from a range of known concentrations of recombinant HERV ENV proteins which allows to obtain a linear equation. In particular, the recombinant HERV ENV proteins are oligomeric recombinant HERV ENV proteins, such as oligomeric recombinant HERV-W ENV proteins, for example hexameric recombinant HERV-W ENV proteins, or oligomeric recombinant HERV-K ENV proteins. Oligomeric recombinant HERV-K ENV may be obtained in vitro from monomeric recombinant HERV-K ENV as further described.

The person skilled in the art knows how to select the appropriate calibration curve depending on the type of HERV ENV protein to be detected. Indeed, in case of the detection of HERV-W ENV, the calibration curve is carried out with known concentrations of recombinant HERV-W ENV proteins, and in case of the detection of HERV-K ENV, the calibration curve is carried out with known concentrations of recombinant HERV-K ENV proteins.

According to the present disclosure "oligomeric" means that HERV ENV proteins may be associated with each other to form a complex of proteins. These may be for example dimers, trimers, tetramers or hexamers. In the serum of patients, HERV ENV proteins are under oligomeric forms. For example, HERV-W ENV is under hexameric form.

The comparison of the electropherogram of the sample to be analyzed with the calibration curve, obtained from electropherograms of known concentrations of HERV ENV protein, allows to quantify the concentration of HERV ENV in the sample to be analyzed.

In more details, the calibration curve is obtained from electropherograms from capillary immunoelectrophoresis of each of the known concentrations of HERV ENV protein. For each known concentration of HERV ENV proteins, an area under the curve (AUC) is obtained on the electropherogram. The relation between the known concentrations of HERV ENV protein and the AUC obtained for each one, gives a linear equation. The calibration curve is then obtained from this linear equation. The electropherogram of a sample to be analyzed provides an AUC. From this AUC, the concentration of the sample can be obtained from the correspondence given by the standard curve.

Of course, the AUC is the AUC of the peak corresponding to the molecular weight of the protein.

The quantitative detection method of HERV ENV in a sample to be analyzed comprises:

- Part 1 comprising:

  • Loading the sample to be analyzed in capillary immunoelectrophoresis,
  • Loading a biotinylated anti-HERV ENV antibody in capillary immunoelectrophoresis,
  • Loading a marker conjugated to streptavidin in capillary immunoelectrophoresis,
  • Detection of the signal of the marker,
  • Obtaining an electropherogram of the signal intensity as a function of the apparent molecular weight of proteins from the sample to be analyzed,
  • Calculating the area under the curve (AUC) of the specific peak corresponding to the immunodetected protein with corresponding molecular weight.

- Part 2 comprising:

  • Loading samples of known concentrations of recombinant HERV ENV protein in capillary immunoelectrophoresis,
  • Loading a biotinylated anti-HERV ENV antibody in capillary immunoelectrophoresis,
  • Loading a marker conjugated to streptavidin in capillary immunoelectrophoresis,
  • Detection of the signal of the marker,
  • Obtaining an electropherogram of each sample of known concentration in recombinant HERV ENV protein,
  • Calculating the area under the curve (AUC) of the specific peak corresponding to the immunodetected recombinant protein of the corresponding molecular weight,

- Establishing a calibration curve with values of AUC from each known concentration of recombinant protein in function of said known concentrations and calculating the equation of the linear range of the curve.

- Part 3 consisting in the determination of the quantity of HERV ENV protein in the sample to be analyzed, which is obtained with the calibration curve, from the measured AUC of the specific electropherogram peak obtained with the sample to be analyzed.

Part 1 and Part 2 can be reversed and can be performed respectively at two separate points in time.
In more details, the quantitative detection method of HERV ENV in a sample to be analyzed comprises:

- Part 1 comprising:

  - Loading the sample to be analyzed in capillary immunoelectrophoresis with a denaturing buffer (sample buffer Ref. from the provider of the simple western platform for immunocapillary analysis, Biotechne, USA),
  - Washing steps before loading the successive compounds and reagents,
  - Fixing the migrated proteins onto the gel present within capillaries by UV irradiation,
  - Loading a biotinylated antibody biotinylated anti-HERV ENV in capillary immunoelectrophoresis,
  - Loading a marker conjugated to streptavidin in capillary immunoelectrophoresis,
  - Detection of the signal generated by a molecule when reacting with the marker, e.g., a chemiluminescence-generating enzyme substrate,
  - Obtaining an electropherogram of the signal intensity as a function of the apparent molecular weight of proteins from the sample to be analyzed, as migrated and fixed along the capillary,
  - Calculating the area under the curve (AUC) of the specific peak corresponding to the immunodetected protein with corresponding molecular weight.

- Part 2 comprising:

  - Loading samples of known concentrations of recombinant HERV ENV protein with an appropriate number of concentrations for a calibration curve, in capillary immunoelectrophoresis
  - Fixing the migrated proteins onto the gel present within capillaries by UV irradiation.
  - Washing steps before loading the successive compounds and reagents,
  - Loading a biotinylated anti-HERV ENV antibody in capillary immunoelectrophoresis,
  - Loading a marker conjugated to streptavidin in capillary immunoelectrophoresis,
  - Detection of the signal generated by a molecule when reacting with the marker, e.g., a chemiluminescence-generating enzyme substrate,
  - Obtaining an electropherogram of each sample of known concentration in recombinant HERV ENV protein,
  - Calculating the area under the curve (AUC) of the specific peak corresponding to the immunodetected recombinant protein of the corresponding molecular weight.
  - Establishing a calibration curve with values of AUC from each known concentration of recombinant protein and calculating the equation of the linear range of the curve

- Part 3 consisting in the determination of the quantity of HERV ENV protein in the sample to be analyzed, which is obtained with the calibration curve, from the measured AUC of the specific electropherogram peak obtained with the sample to be analyzed.

Part 1 and Part 2 can be reversed and can be performed respectively at two separate points in time.

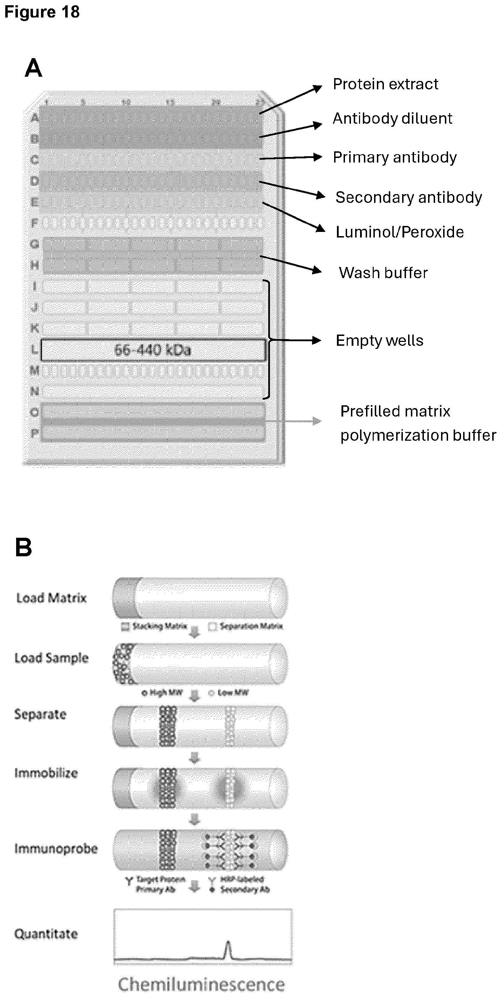

Figure 18 is a general illustration of the detection of an antigen by capillary immunoelectrophoresis.

***Oligomerization of recombinant HERV ENV proteins and calibration curve***

The calibration curve is obtained from a range of known concentrations of recombinant HERV ENV proteins which allows to obtain a linear equation. In particular, the recombinant HERV ENV proteins are oligomeric proteins, such as hexameric recombinant HERV-W ENV proteins. They may be obtained by incubating monomeric recombinant HERV ENV proteins in a solubilization buffer.
The incubation is between 20 and 40 min, 25 and 35 min, for example 30 min.
Typically, the solubilization buffer comprises:

- Bicine (pH7.5 buffer)

- A zwitterionic detergent, such as CHAPS, 3-[(3-cholamidopropyl)dimethylammonio]-1-propanesulfonate, $C_{32}H_{58}N_2O_7S$) and/or a detergent with a polar extremity and a long aliphatic chain, such as LPG [Lyso PG 1-myristoyl-2-hydroxy-sn-glycero-3-phospho-(1'-rac-glycerol)] or fos-Choline 16 ( n-Hexadecylphosphocholine, C21 H46NO4P)
- Optionally a dilution or a protein extract from serum, such as human serum or fetal calf serum,
- A protease inhibitor, or a mix of protease inhibitors.

The protease inhibitor may be an inhibitor of serine proteases, aminopeptidase B, Leucine aminopeptidase, cysteine proteases, trypsin-like proteases, aspartic proteases, or a mix thereof. The protease inhibitor is for example: 4-(2-Aminoethyl)benzenesulfonyl fluoride hydrochloride (AEBSF hydrochloride), aprotinin such as aprotinin from bovine lung, bestatin, E-64, leupeptin, also called leupeptin hemisulfate, pepstatin A, or a mix thereof.

AEBSF hydrochloride and aprotinin are inhibitors of serine proteases, bestatin is an inhibitor of aminopeptidase B and Leucine aminopeptidase, E-64 and leupeptin are inhibitors of cysteine proteases, pepstatin A is an inhibitor of aspartic proteases.

When it is present in the solubilization buffer, the protein extract from serum is at a final concentration in-between 1 to 90%.

For example, the solubilization buffer can be obtained from the Mem-PER plus membrane protein extraction kit, reference 89842, Thermoscientific.

Indeed, unexpected results were observed using the buffer of the Mem-PER protein extraction kit, under conditions that differed from the recommended use of this kit, among the various chemical compounds of buffers tested with this protein. When purified HERV-W ENV recombinant protein was diluted in the "solubilization buffer" from the "Mem-PER kit" and incubated for a few minutes at room temperature, a fully oligomerized HERV-W ENV has been obtained, corresponding to the antigenic form found in human biological fluids, such as serum, without significant remains of the incubated recombinant monomer.

The present application also deals with a process for in vitro oligomerization of recombinant HERV ENV proteins comprising the incubation of monomeric recombinant HERV ENV proteins in a solubilization buffer as above described.

For example, the incubation is between 20 and 40 min, 25 and 35 min, for example 30 min.

In particular, it is a process for in vitro oligomerization, in particular hexamerization, of recombinant HERV-W ENV proteins comprising the incubation of monomeric recombinant HERV-W ENV proteins in a solubilization buffer as above described.

In particular, it is a process for in vitro oligomerization of recombinant HERV-K ENV proteins comprising the incubation of monomeric recombinant HERV-K ENV proteins in a solubilization buffer as above described.

Thus, the present application also deals with the use of a solubilization buffer as above described to obtain oligomers of recombinant HERV-K ENV proteins from monomeric recombinant HERV-K ENV proteins.

The present application also deals with an in vitro process for obtaining oligomeric recombinant HERV ENV proteins comprising:

- Expression of monomeric recombinant HERV ENV protein in E. coli,
- Purification of the monomeric recombinant HERV ENV proteins obtained from E. coli,
- Incubation in a solubilization buffer as above described for oligomerization.

For example, the incubation is between 20 and 40 min, 25 and 35 min, for example 30 min.

In particular, it is an in vitro process for obtaining oligomeric recombinant HERV-W ENV proteins, in particular hexameric recombinant HERV-W ENV proteins, comprising:

- Expression of monomeric recombinant HERV-W ENV protein in E. coli, in particular the recombinant HERV-W ENV protein of SEQ ID NO:16, for example encoded by the nucleic acid of SEQ ID NO:15;
- Purification of the monomeric recombinant HERV-W ENV protein obtained from E. coli,
- Incubation in a solubilization buffer as above described to obtain oligomeric HERV-W ENV proteins, in particular hexameric HERV-W ENV proteins.

In particular, it is an in vitro process for obtaining oligomeric recombinant HERV-K ENV proteins comprising:

- Expression of monomeric recombinant HERV-K ENV protein in E. coli, in particular the recombinant HERV-K ENV protein of SEQ ID NO:22, for example encoded by the nucleic acid of SEQ ID NO:21;
- Purification of the monomeric recombinant HERV-K ENV protein obtained from E. coli,
- Incubation in a solubilization buffer as above described to obtain oligomeric HERV-K ENV proteins.

The present application also relates to a method for establishing a calibration curve of oligomeric recombinant HERV ENV proteins comprising:

- Obtaining oligomeric recombinant HERV ENV proteins as above described,
- Establishing a range of known concentrations in oligomeric recombinant HERV ENV proteins,
- Loading samples of known concentrations of recombinant HERV ENV protein in capillary immunoelectrophoresis,
- Loading a biotinylated anti-HERV ENV antibody in capillary immunoelectrophoresis,
- Loading a marker conjugated to streptavidin in capillary immunoelectrophoresis,
- Detection of the signal of the marker,
- Obtaining an electropherogram of each sample of known concentration in recombinant HERV ENV protein,
- Calculating the area under the curve (AUC) of the specific peak corresponding to the immunodetected recombinant protein of the corresponding molecular weight,
- Establishing a calibration curve with values of AUC from each known concentration of recombinant protein in function of said known concentrations and calculating the equation of the linear range of the curve.

The person skilled in the art knows how to determine an appropriate number of concentrations for a calibration curve.

In particular, this is a method for establishing a calibration curve of oligomeric recombinant HERV-W ENV proteins or oligomeric recombinant HERV-K ENV proteins.

The present application also deals with HERV-K ENV protein of SEQ ID NO:22, for example encoding by the nucleic acid of SEQ ID NO:21.

A highly hydrophobic C-term extremity is required for HERV protein auto-assembly, i. e. oligomerization. However, HERV-K ENV does not possess hydrophobic domain at its C-term extremity. The inventors have modified the reading frame in order to replace the regular extremity of HERV ENV with low hydrophobicity, by a highly hydrophobic alternative (ALT) polypeptide at its C-terminal extremity, in order to render possible the in vitro oligomerization.

It also relates to the use of a HERV-K ENV protein of SEQ ID NO:22 to establish a calibration curve, i.e. a linear equation, of the concentration of HERV ENV proteins in function of the AUC obtained on the electropherogram in capillary immunoelectrophoresis.

The present application also deals with a series of vials comprising a range of known concentrations, one vial by concentration, of oligomeric recombinant HERV ENV proteins, in particular HERV-W ENV such as protein of sequence SEQ ID NO: 16 or HERV-K ENV, such as protein of sequence SEQ ID NO: 22.

The series of vials may be used to establish a calibration curve of oligomeric recombinant HERV ENV proteins.

### Preparation of the sample

In particular, prior to its analysis, the sample to be analyzed is prepared as follows:

    a. Incubation with a permeabilization buffer
    b. Removal of supernatant after centrifugation
    c. Addition of a solubilization buffer
    d. Incubation
    e. Recovery of supernatant after centrifugation

In particular, incubation at step a) is between 5 and 20 min, 5 and 15 min, for example 10 min. The ratio sample/permeabilization buffer is 1:10 v/v. The permeabilization buffer comprises a Bicine buffer or tris buffer pH7.5, a detergent such as Tween 20 (monolaurate de polyoxyethylene sorbitane) or NP40 (octylphenoxypolyethoxyethanol), and a protease inhibitor or a mix of protease inhibitors. The protease inhibitor may be an inhibitor of serine proteases, aminopeptidase B, Leucine aminopeptidase, cysteine proteases, trypsin-like proteases, aspartic proteases, or a mix thereof. The protease inhibitor is for example: 4-(2-Aminoethyl)benzenesulfonyl fluoride hydrochloride (AEBSF hydrochloride), aprotinin such as aprotinin from bovine lung, bestatin, E-64, leupeptin, also called leupeptin hemisulfate, pepstatin A, or a mix thereof.

At step b, the centrifugation is for example between 10 and 25 min, 10 and 20 min, for example 15 min at 14 000 g.

The solubilization buffer at step c is as described above. It is added to the pellet retrieved after removing the supernatant. In particular 100µl of solubilization buffer are added.

Incubation at step d is between 20 and 40 min, 25 and 35 min, for example 30 min.

At step e, the centrifugation is for example between 10 and 25 min, 10 and 20 min, for example 15 min at 14 000 g.

Centrifugation steps are performed at room temperature.

For example, the permeabilization buffer and the solubilization buffer come from the Mem-PER plus membrane protein extraction kit, reference 89842, Thermoscientific.

Thus, the present disclosure also deals with a method of preparation of a sample, in particular serum, which comprises an incubation with a permeabilization buffer and an addition of a solubilization buffer. Details of the buffers are provided above.

In particular, this preparation is for a detection method of HERV ENV, in particular a quantitative detection method of HERV ENV, for example by capillary immunoelectrophoresis in particular with an anti-HERV ENV biotinylated antibody.

### Anti-HERV ENV biotinylated antibody

According to another aspect, the present disclosure also relates to anti-HERV ENV biotinylated antibody.

For example, said anti-HERV ENV biotinylated antibody is an anti-HERV-W ENV biotinylated antibody. In particular, it is an anti-HERV-W ENV biotinylated antibody which comprises each of the complementary-determining regions (CDRs) set forth in SEQ ID NO: 1, SEQ ID NO: 2, SEQ ID NO: 3 SEQ ID NO: 4, SEQ ID NO: 5 and SEQ ID NO: 6.

More particularly, said anti-HERV-W ENV biotinylated antibody comprises:

- a light chain comprising a light chain variable region (VL) which comprises three complementarity determining regions: CDR-L1 as set forth in SEQ ID NO:1, CDR-L2 as set forth in SEQ ID NO:2 and CDR-L3 as set forth in SEQ ID NO:3, and

- a heavy chain comprising a heavy chain variable region (VH) which comprises three complementarity determining regions: CDR-H1 as set forth in SEQ ID NO:4, CDR-H2 as set forth in SEQ ID NO:5 and CDR-H3 as set forth in SEQ ID NO:6.

In particular, the HERV-W ENV biotinylated antibody is a humanized biotinylated antibody.

More particularly, said humanized HERV-W ENV biotinylated antibody comprises:

- a light chain comprising a light chain variable region (VL) as depicted in SEQ ID NO:7, and

- a heavy chain comprising a heavy chain variable region (VH) as depicted in SEQ ID NO:8.

For example, the anti-HERV-W ENV biotinylated antibody is Temelimab.

For example, said anti-HERV ENV biotinylated antibody is an anti-HERV-K ENV biotinylated antibody.

In particular, it is an anti-HERV-K ENV biotinylated antibody which comprises each of the complementary-determining regions (CDRs) set forth in SEQ ID NO: 9, SEQ ID NO: 10, SEQ ID NO: 11 SEQ ID NO: 12, SEQ ID NO: 13 and SEQ ID NO: 14.

More particularly, said anti-HERV-K ENV biotinylated antibody comprises:

- a light chain comprising a light chain variable region (VL) which comprises three complementarity determining regions: CDR-L1 as set forth in SEQ ID NO:9, CDR-L2 as set forth in SEQ ID NO:10 and CDR-L3 as set forth in SEQ ID NO:11, and

- a heavy chain comprising a heavy chain variable region (VH) which comprises three complementarity determining regions: CDR-H1 as set forth in SEQ ID NO:12, CDR-H2 as set forth in SEQ ID NO:13 and CDR-H3 as set forth in SEQ ID NO:14.

In particular, the anti-HERV-K ENV biotinylated antibody is a humanized biotinylated antibody.

The present disclosure also relates to the use of anti-HERV ENV biotinylated antibody as above described in a detection method of HERV ENV, in particular in a quantitative detection method of HERV ENV.

The detection method may be by capillary immunoelectrophoresis.

It is a detection method of HERV-W ENV or HERV-K ENV depending on the anti-HERV ENV biotinylated antibody used.

### Kit for detection method of HERV ENV

According to another aspect, the present disclosure relates to a kit for a detection method of HERV ENV.

The present application also relates to a kit comprising:

- An anti-HERV ENV biotinylated antibody as above described, and

- A marker conjugated to streptavidin.

In particular, it is a kit for a quantitative detection method of HERV ENV.
In particular, the kit further comprises a series of vials comprising a range of known concentrations of oligomeric recombinant HERV ENV proteins, as previously described. This is for the user to establish the calibration curve.
The kit is for detecting HERV-W ENV or HERV-K ENV depending on the anti-HERV ENV biotinylated antibody in the kit.

**Table 1: CDRs sequences**

| antibody HERV-W ENV CDR1 | SEQ ID NO:1 | SASSSVSYMY |
|---|---|---|
| antibody HERV-W ENV CDR2 | SEQ ID NO:2 | RTSNLAS |
| antibody HERV-W ENV CDR3 | SEQ ID NO:3 | QQYQSLPLT |
| antibody HERV-W ENV CDR4 | SEQ ID NO:4 | DYEMH |
| antibody HERV-W ENV CDR5 | SEQ ID NO:5 | AVAPETGGTA YNQKFKG |
| antibody HERV-W ENV CDR6 | SEQ ID NO:6 | TVVPFAY |
| antibody HERV-W ENV_VL | SEQ ID NO:7 | QIQLTQSPSSLSASVGDRVTITCSASS SVSYMYWYQQKPGKAPKAWIYRTSNL ASGVPSRFSGSGSGTDYTLTISSLQPE DFATYYCQQYQSLPLTFGGGTKVEIK |
| antibody HERV-W ENV_VH | SEQ ID NO:8 | QVQLVQSGAEVKKPGSSVKVSCKASG YTFTDYEMHWVRQAPGQGLEWIGAVA PETGGTAYNQKFKGRATITADKSTSTA YMELSSLRSEDTAVYYCTSTVVPFAYW GQGTLVTVSS |
| antibody HERV-K ENV CDR1 | SEQ ID NO:9 | QSLLDSDGKT Y |
| antibody HERV-K ENV CDR2 | SEQ ID NO:10 | LVS |
| antibody HERV-K ENV CDR3 | SEQ ID NO:11 | LQATHFPWT |
| antibody HERV-K ENV CDR4 | SEQ ID NO:12 | GYTFTSYW |
| antibody HERV-K ENV CDR5 | SEQ ID NO:13 | IDPYDSET |
| antibody HERV-K ENV CDR6 | SEQ ID NO:14 | ASLYYYGISL |

**FIGURES LEGENDS**

**Figure 1:** Classical HERV-ENV protein structure

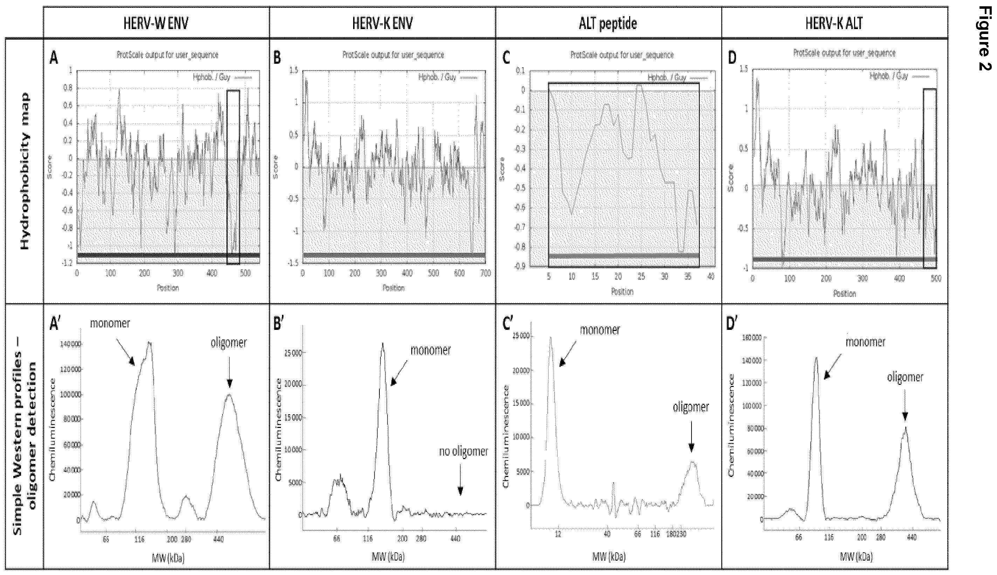

**Figure 2: HERVs protein oligomerization is dependent on the presence of a hydrophobic extremity.** Hydrophobic amino acid mapping was performed using "Guy's hydrophobic score calculation" along the amino acid sequence of HERV-W ENV **(A),** HERV-K ENV **(B),** ALT polypeptide **(C)** and the frameshifted protein HERV-K ALT **(D).** Guy's score > 0 reveals hydrophilic amino acids and residues obtaining a score < 0 are hydrophobic (**brown areas**). Hydrophobic domains are highlighted on each graph using a grey square. Electrophoregrams obtained by Simple Western with lysates of transfected HEK293T cells were analyzed in the high molecular weight part of the separation matrix. HERV-W ENV was detected with GN_mAb_Env01 antibody (**A'**), HERV-K ENV and HERV-K ALT (**B'** and **D',** respectively) were detected with GN_mAb_Env-K01 antibody, ALT polypeptide was detected using an anti-FLAG tag pAb **(C').**

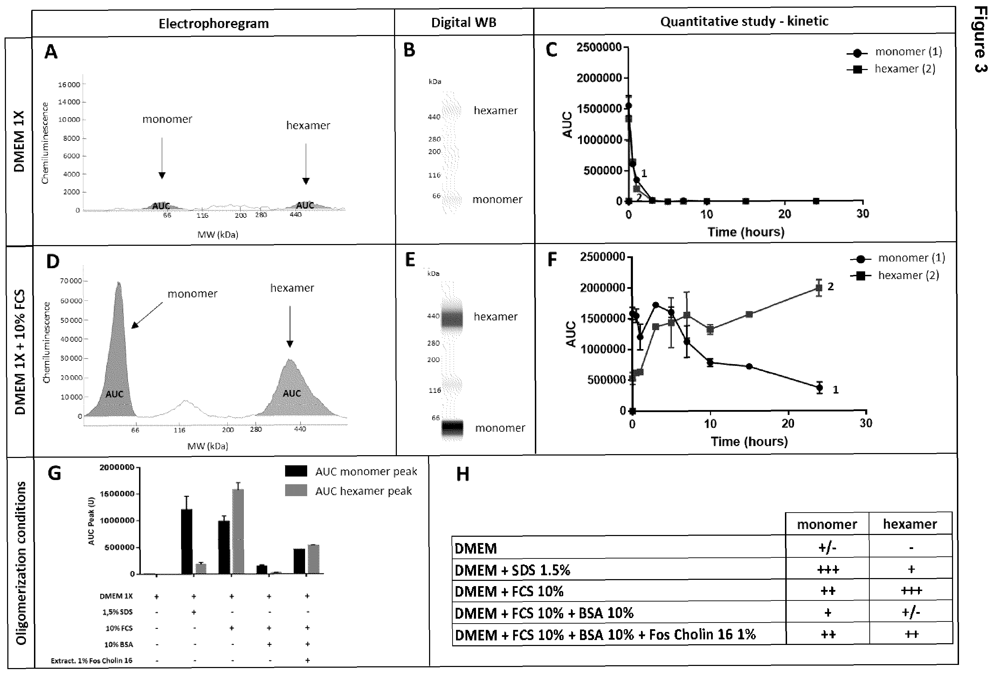

**Figure 3: HERV envelope oligomers can partially be extracted from biological media in presence of a specific detergent.** Automated capillary western blot technology (Simple Western) was used for characterizing HERV-W ENV antigen produced in E. coli expressing system (no post translational modification). HERV-W ENV detection was performed with GN_mAb_Env01 antibody. This highly sensitive technology provided different types of information: migration electrophoregrams (**A, D**), digital western blots (**B, E**) and quantitative interpretation based on calculation of the area under curve "AUC" (**C, F** and **G**). Here, the self-assembly properties of HERV-W ENV were assessed in basic DMEM or DMEM media with 10% foetal calf serum (FCS) (**A-F**). Kinetics of oligomerization were performed within 24h of incubation, supernatants were harvested at different time-points and each form of HERV-W ENV was quantified (**C, F**). HERV-W ENV oligomerization characteristics after overnight (ON) incubation at 37°C in presence of 1.5% SDS, 10% FCS, 10% FCS + 10% BSA extracted without fos choline 16, or with fos choline 16 when mentioned, are documented in G and H.

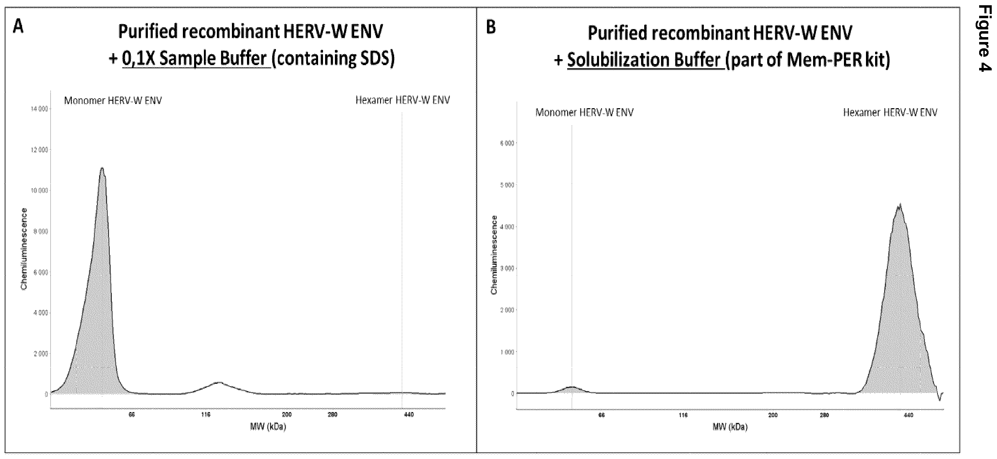

**Figure 4: Solubilization buffer from Mem-PER protein extraction kit induces oligomerization of HERVs proteins (example of HERV-W ENV).** Purified HERV-W ENV monomer (58kDa) was diluted in 0.1X classical Sample Buffer for Simple Western (**A**) or in Solubilization buffer from Mem-PER protein extraction kit (**B**). Unlike in 0.1X classical Sample Buffer for Simple Western, where HERV-W ENV remained under antigenic monomer form (**A**), this protein oligomerised to antigenic hexamer form (400-440kDa) when it was diluted in Solubilization buffer from Mem-PER protein extraction kit (**B**).

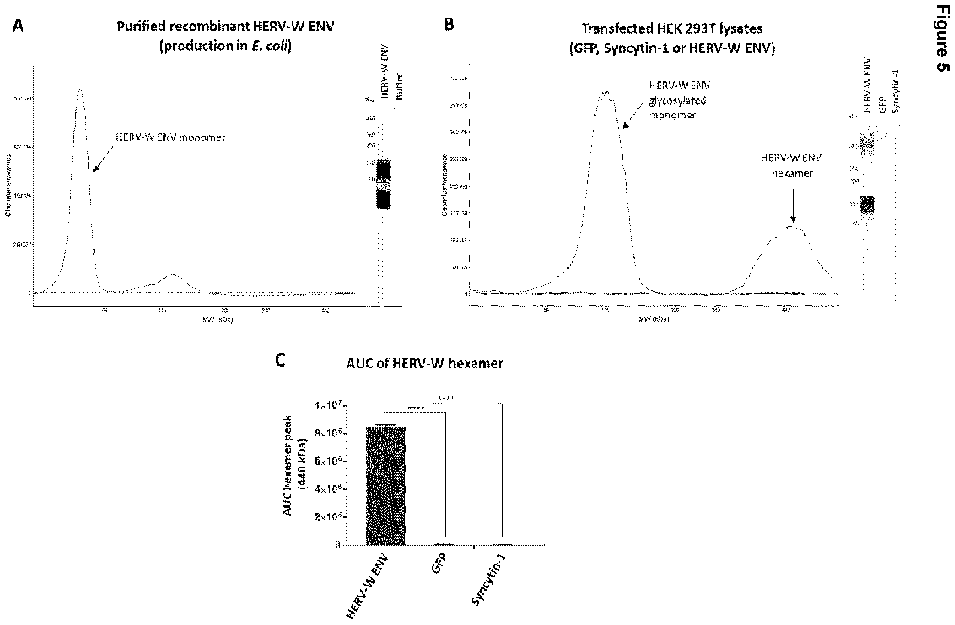

**Figure 5: Simple Western profile of HERV-W ENV detection by GN_mAb_Env01.** (**A**) Elecrophoregram and digital western blot representation of detection signal using recombinant HERV-W ENV protein produced in procaryote system or buffer as blank. (**B**) Same representation using human transfected cell lysate respectively expressing GFP (negative control), Syncytin-1 (specificity control) or HERV-W ENV (positive control). (**C**) Histograms reprensenting quantification of signal correction to HERV-W ENV hexamer detected in human transfected cell lysate respectively expressing GFP, Syncytin-1 or HERV-W ENV.

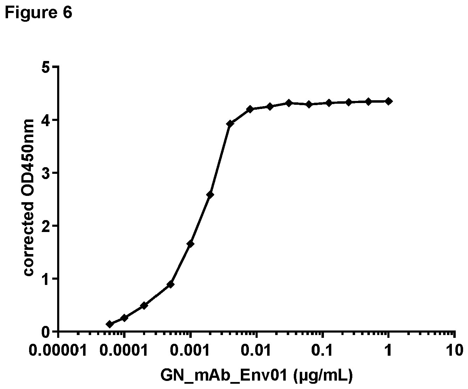

**Figure 6: Direct ELISA results.** Corrected Optic Density (OD) measured at 450 nm dependent of GN_mAb_Env01. Corrected OD consist of measured OD minus OD measured in blank well (0µg/mL GN_mAb_Env01).

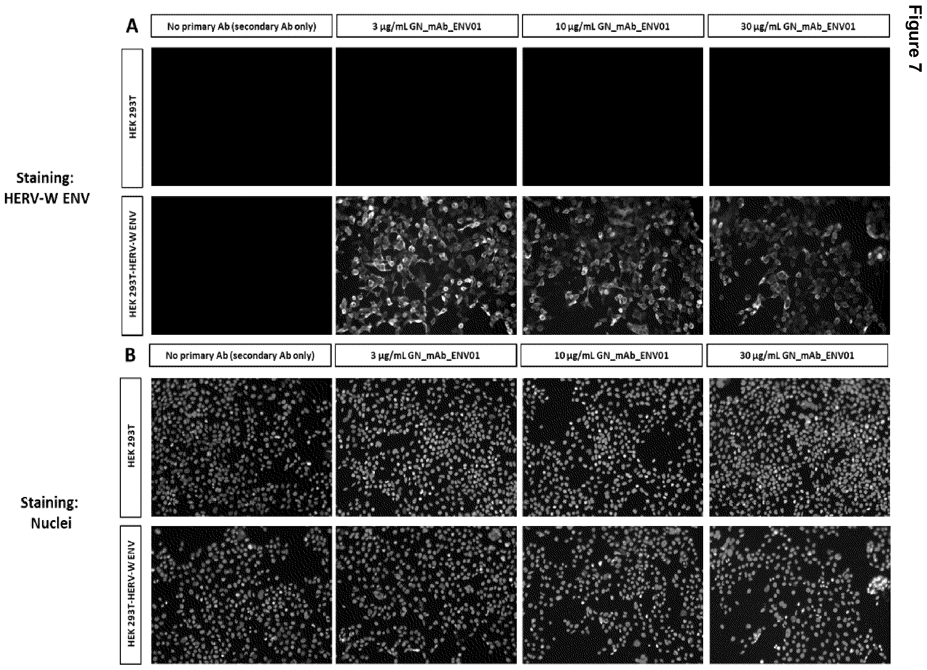

**Figure 7: HERV-W ENV immunostaining on transfected cells.** (**A**) The upper line of pictures represents immunofluorescent results obtained with or without GN_mAb_Env01 as primary antibody (see detail in column title) on non-transfected cells (blank). The lower line showed the results obtained on HERV-ENV transfected cells (positive control). (**B**) Nuclei staining with DAPI revealing all cells present in the microscopic field.

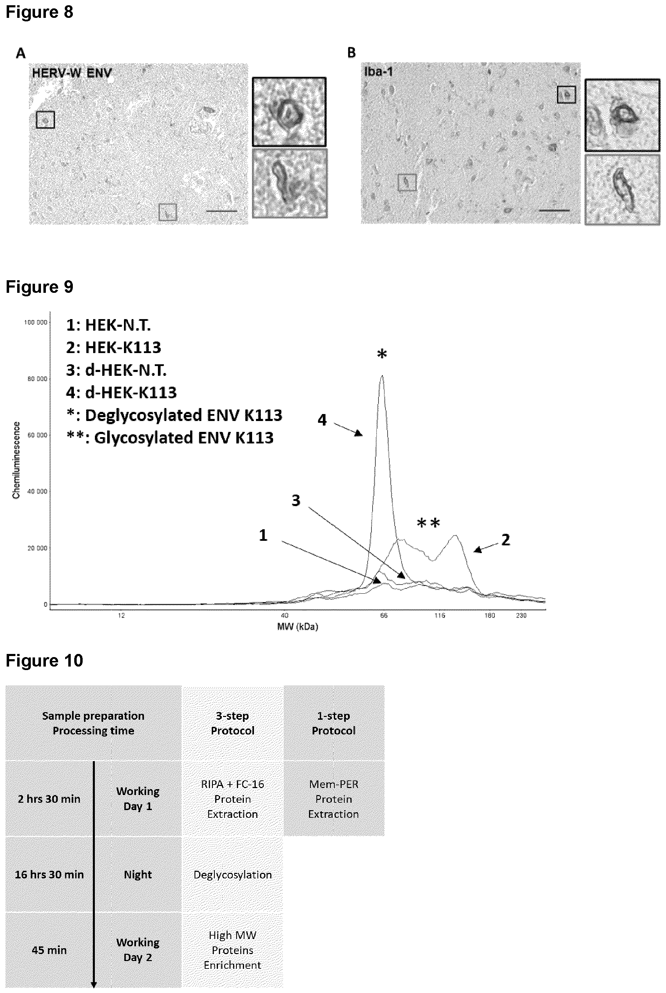

**Figure 8: HERV-W ENV immunostaining (IHC) on human brain necropsies from COVID-19 patients.** Detection of HERV-W in microglial cells was assessed using anti-HERV-W ENV (**A**) and anti-microglia specific marker Iba-1 (**B**) in adjacent sections of brain parenchyma. The morphology of HERV-W ENV positive cells is highlighted with higher magnification of small round cells (**A**, black square) and small elongated cells (**A**, grey square). Higher magnifications of Iba-1 positive small round (**B**, black square) and small elongated cells (**B**, grey square) are also boxed. Bars = 100µm.

**Figure 9: GN_mAb_Env-K01 antibody characterization by Simple Western on glycosylated and deglycosylated HERV-K ENV.** Presented data at Figure 9 were obtained with 1500 µg/mL non-transfected cell lysate (1, HEK-N.T.), deglycosylated non transfected cell lysate (3, d-HEK-N.T.), pCMV-HERV-K113 transfected cell lysate (2, HEK-K113) or deglycosylated pCMV-HERV-K113 transfected cell lysate (4, d-HEK-K113) and 50 µg/mL of GN_mAb_Env-K01.

**Figure 10: Schematic representation of the duration of the 2 sample preparation protocols used for HERV-W ENV detection in human serum.** The 3-step protocol used to prepare human serum sample for HERV-W ENV detection lasts approximately 20 hours while the 1-step protocol lasts 2 hours and a half.

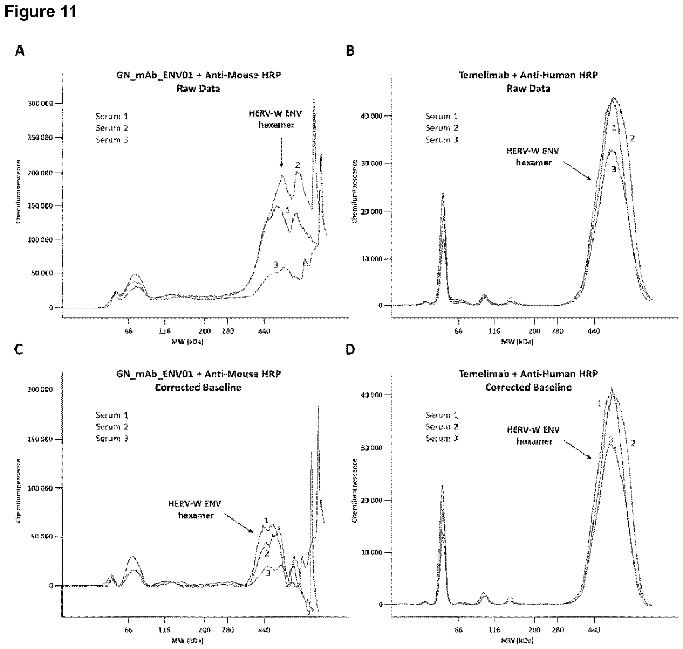

**Figure 11: Improvement of HERV-W ENV hexamer peak definition.** 3 human sera were prepared according to 3-step protocol followed by Simple Western analysis using 20 µg/mL murine monoclonal GN_mAb_Env01 antibody as primary antibody and anti-mouse HRP as secondary antibody (**A, C**) or according to 1-step protocol followed by Simple Western analysis using 20 µg/mL Temelimab as primary antibody and anti-human HRP as secondary antibody (**B, D**). Electrophoregrams before (**A, B**) or after baseline adjustment (**C, D**). Black arrows indicate HERV-W ENV hexamer peak (≈ 400-440 kDa).

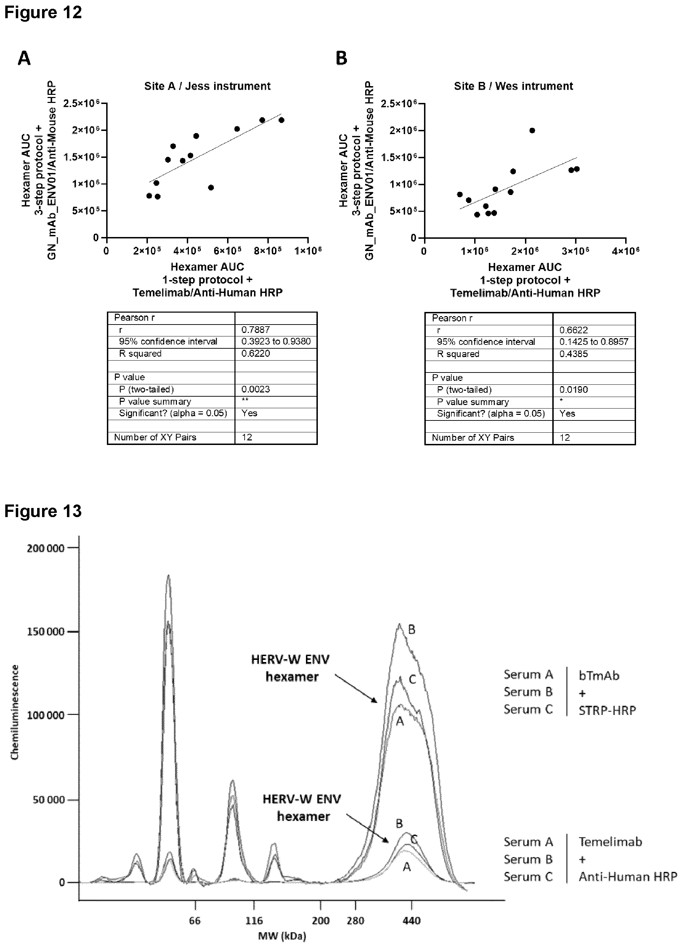

**Figure 12: Correlation of HERV-W ENV detection using 3-step test and GN_mAb_Env01 as primary antibody or 1-step test and Temelimab as primary antibody.** 12 serums were prepared and analyzed in parallel on site A with Simple Western device Jess (**A**) or on site B with Wes instrument (**B**). The strength of correlation was assessed using Pearson r test.

**Figure 13: Assay sensitivity is optimized using bTmAb + STRP-HRP as detection method.** 3 human sera were prepared according to 1-step test and analyzed by Simple Western either with 20 $\mu$g/mL Temelimab and anti-human HRP secondary antibody or with 20 $\mu$g/mL bTmAb and Streptavidin-HRP reagent.

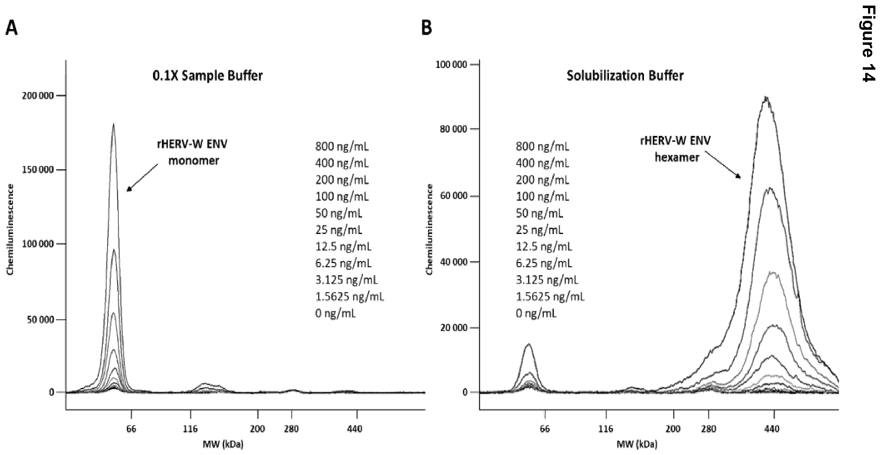

**Figure 14: Hexamerisation of recombinant HERV-W ENV in Solubilization Buffer.** 11 points recombinant HERV-W ENV range starting from 800 ng/mL (two-fold serial dilution) was prepared in 0.1X Sample Buffer **(A)** or in Solubilization Buffer **(B).** Recombinant HERV-W ENV monomer peak **(A)** and hexamer peak **(B)** are indicated by black arrows on electrophoregrams.

**Figure 15: Recombinant HERV-W ENV hexamer standard curve.** 440 kDa peakAUC was measured for each recombinant HERV-W ENV range point and the resulting recombinant HERV-W ENV hexamer standard curve was analyzed using linear regression (12.5 to 200 ng/mL) **(A)** or non-linear logarithmic fit (3.125 to 400 ng/mL) **(B). (C)** Standard curve recovery percentages were calculated for the two analytical fits. **(D)** Hexamer LOD and LOQ values were determined at 2.20 and 8.05 ng/mL respectively.

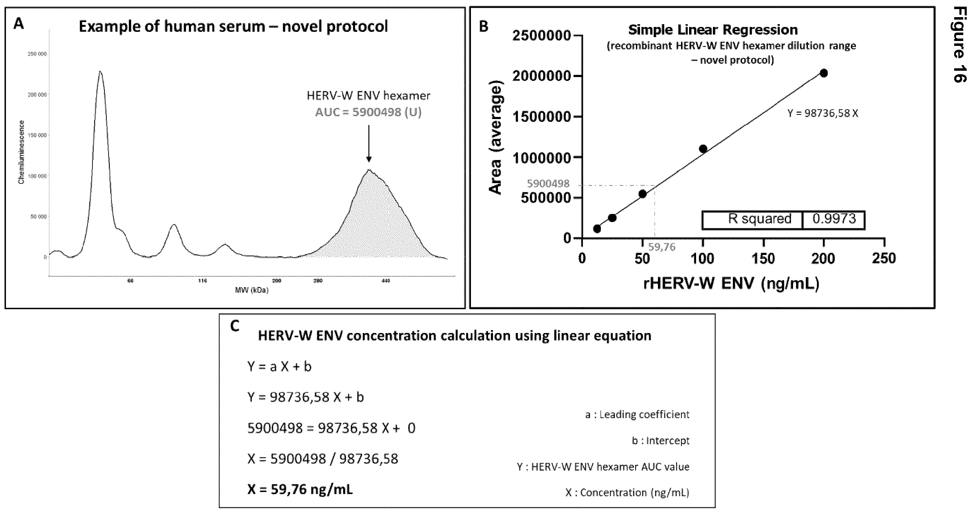

**Figure 16: Example of absolute HERV-W ENV hexamer quantification in human serum:** Human serum was prepared following the novel sample preparation protocol and the electrophoregram obtained after Simple Western analysis **(A).** Thanks to the dilution range of hexameric HERV-W ENV obtained by incubation of recombinant HERV-W ENV monomer in the solubilization buffer from Mem-PER kit, a linear equation can be determined **(B).** Applying the AUC value measured on electrophoregram into linear equation, the absolute quantity or HERV-W ENV hexamer contained in the human serum can be determined **(C).**

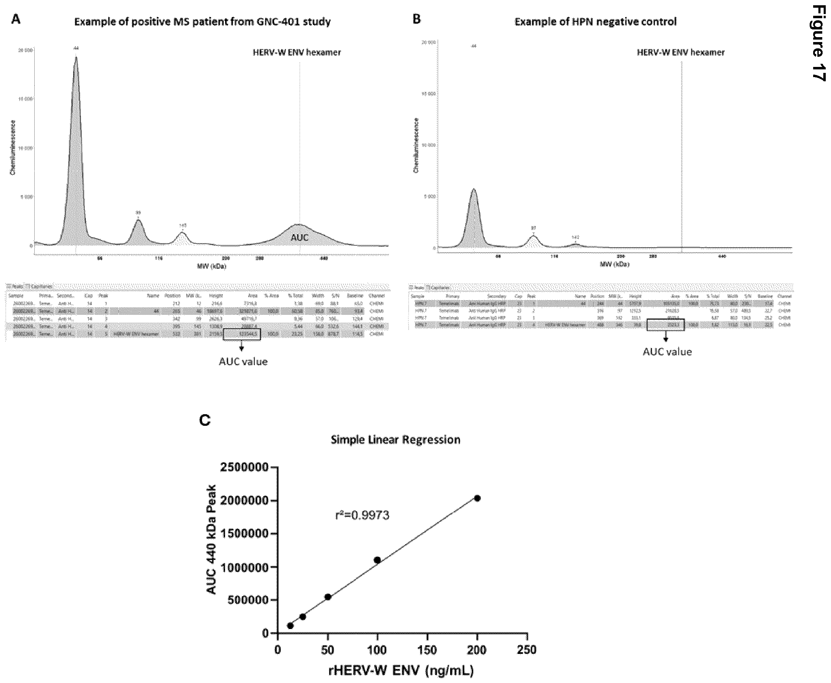

**Figure 17: Simple Western profile examples of positive and negative CSF for HERV-W ENV:** Simple Western electrophoregram profile obtained on positive **(A)** and negative **(B)** CSF for HERV-ENV. Proteins present in the Sample were separated in separation matrix depending of their molecular weight (MW). After detection using temelimab, the area under curve (AUC) of the peak corresponding to 350-440kDa is recorded in the summary table located under the electrophoregram (black square). AUC value is proportional to the quantity of HERV-W ENV in the sample. **(C)** Standard curve obtained by recombinant protein dilution range used for absolute quantification.

**Figure 18: Illustration of an antigen detection by capillary electrophoresis. A.** Example of organization of the different reagents. **B.** Diagram of the steps leading up to the signal detection.

## EXEMPLES

### EXAMPLE 1: Production and purification of full-length HERV-W ENV protein.

Sequences:

Nucleotides sequence of HERV-W ENV ended with a 6-Histidine tag sequence and a stop codon.

SEQ ID NO:15

atggccctcccttatcatactttctctttactgttctcttacccccttcgctctcactgcaccccctccatgctgctgtacaaccagtagctccccttacc
aagagtttctatggagaacgcggcttcctggaaatattgatgccccatcatataggagtttatctaagggaaactccaccttcactgcccacaccc
atatgccccgcaactgctataactctgccactctttgcatgcatgcaaatactcattattggacagggaaaatgattaatcctagttgtcctggagg
acttggagccactgtctgttggacttacttcacccataccagtatgtctgatggggggtggaattcaaggtcaggcaagagaaaaacaagtaaag
gaagcaatctcccaactgacccgggggacatagcacccctagcccctacaaaggactagttctctcaaaactacatgaaaccctccgtaccca
tactcgcctggtgagcctatttaataccaccctcactcggctccatgaggtctcagcccaaaaccctactaactgttggatgtgcctcccctgcac
ttcaggccatacatttcaatccctgttcctgaacaatggaacaacttcagcacagaaataaacaccacttccgttttagtaggacctcttgtttccaa
tctggaaataacccatacctcaaacctcacctgtgtaaaatttagcaatactatagacacaaccagctcccaatgcatcaggtgggtaacacct
cccacacgaatagtctgcctaccctcaggaatattttttgtctgtggtacctcagcctatcattgtttgaatggctcttcagaatctatgtgcttcctctca
ttcttagtgcccctatgaccatctacactgaacaagatttatacaatcatgtcgtacctaagccccacaacaaaagagtacccattcttccttttgtt
atcagagcaggagtgctaggcagactaggtactggcattggcagtatcacaacctctactcagttctactacaaactatctcaagaaataaatg
gtgacatggaacaggtcactgactccctggtcaccttgcaagatcaacttaactccctagcagcagtagtccttcaaaatcgaagagctttagac
ttgctaaccgccaaaagagggggaacctgtttattttttaggagaagaacgctgttattatgttaatcaatccagaattgtcactgagaaagttaaa
gaaattcgagatcgaatacaatgtagagcagaggagcttcaaaacaccgaacgctggggcctcctcagccaatggatgccctggactctccc
cttcttaggacctctagcagctataatattttttactcctctttggaccctgtatcttcaacttccttgttaagtttgtctcttccagaattgaagctgtaaagct
acaaatagttcttcaaatggaaccccagatgcagtccatgactaaaatctaccgtggaccccctggaccggcctgctagactatgctctgatgtta
atgacattgaagtcacccctcccgaggaaatctcaactgcacaaccccctactacactccaattcagtaggaagcagt*catcatcatcatcatcat*
TAG

Amino acids sequence of the recombinant his-tagged HERV-W ENV protein

SEQ ID NO:16

MALPYHTFLFTVLLPPFALTAPPPCCCTTSSSPYQEFLWRTRLPGNIDAPSYRSLSKGNSTFTAHTHM
PRNCYNSATLCMHANTHYWTGKMINPSCPGGLGATVCWTYFTHTSMSDGGGIQGQAREKQVKEAIS
QLTRGHSTPSPYKGLVLSKLHETLRTHTRLVSLFNTTLTRLHEVSAQNPTNCWMCLPLHFRPYISIPVP
EQWNNFSTEINTTSVLVGPLVSNLEITHTSNLTCVKFSNTIDTTSSQCIRWVTPPTRIVCLPSGIFFVCG
TSAYHCLNGSSESMCFLSFLVPPMTIYTEQDLYNHVVPKPHNKRVPILPFVIRAGVLGRLGTGIGSITTS
TQFYYKLSQEINGDMEQVTDSLVTLQDQLNSLAAVVLQNRRALDLLTAKRGGTCLFLGEERCYYVNQ
SRIVTEKVKEIRDRIQCRAEELQNTERWGLLSQWMPWTLPFLGPLAAIIFLLLFGPCIFNFLVKFVSSRIE
AVKLQIVLQMEPQMQSMTKIYRGPLDRPARLCSDVNDIEVTPPEEISTAQPLLHSNSVGSS*HHHHHH*

<u>Production Protocol</u>

Recombinant his-tagged HERV-W ENV (full-length MSRV envelope protein of 548aa; 61,4kDa; ENV pV14; GenBank accession no. AF331500) was produced by GTP Bioways (Toulouse, France) according to quality control specifications of GeNeuro (Geneva, Switzerland). Briefly the following steps were performed:

- Design of pGPTc502-HERV W-6His expression vector and transformation of E. coli bacteria BL21 T1R (DE3) strain
- Expression of the protein in 3Liters of culture of transformed bacteria
- Monitoring of bacteria growth and harvesting
- Cells lysis
- Solubilization
- Purification on IMAC excel resin followed by gel filtration chromatography
- SDS-PAGE analysis
- Protein quantity was measured by UV assay at 280nm using Nanodrop
- Endotoxin-free conditions, Endotoxin assay and, if needed, protein polishing. Endotoxin levels for HERV-W batches were measured using Charles River Endosafe nexgen-PTS (Portable Endotoxin Testing System) test. If the level of endotoxin is >20EU/mL, an Endotoxin removal step must be performed. Endotoxin removal was done by polishing batches through Mustang Q Acrodisc followed by filtration on 0.22μm filter Stericup (Merck, Darmstadt, Germany).

Storage buffer: HERV-W ENV protein was stored in buffer containing 20 mM Tris pH8, 150 mM NaCl, 1.5% SDS, 5 mM DTT, 0.5 M Urea.

**EXAMPLE 2: Production and purification of full-length and frameshifted HERV-K ENV proteins *(SU, SU-TM, SU-ALT and ALT respectively)***

EXAMPLE 2.1. Production of HERV-K ENV (SU) recombinant protein:

Sequences:

Nucleotides sequence of HERV-K ENV with a 6-Histidine tag sequence and a stop codon

SEQ ID NO:17
atg*catcatcatcatcatcat*ctgccgatgccggccggtgcagcagcagcaaattatacctattgggcctatgttccgtttccgccgctgattcgcg
ccgtgacctggatggataatccgaccgaagtgtatgtgaatgatagcgtgtgggttccgggtccgattgatgatcgttgcccggcaaaaccgga
agaagaaggcatgatgattaatattagcattggctatcactacccgccgatttgcctgggtcgtgccccgggttgcctgatgccggctgttcagaa
ttggctggtggaagtgccgaccgtgagtccgatttgtcgttttacctatcatatggttagcggtatgagtctgcgcccgcgcgttaattatctgcagga
ttttagttatcagcgcagcctgaaatttcgcccgaaaggtaaaccgtgtccgaaagaaattccgaaagaaagtaaaaataccgaagttctggtg
tgggaagaatgtgtggcaaatagtgccgttattctgcagaataatgaatttggcaccattattgattgggcaccgcgtggtcagtttatcataattgc
agtggtcagacccagagttgcccgagtgcacaggtgagtccggccgtggatagtgatctgaccgaaagcctggataaacataaacataaaa
agctgcagagctttatccgtgggaatggggtgaaaaaggtattagcaccccgcgtccgaaaattgttagtccggtgagcggcccggaacatc
cggaactgtggcgcctgaccgttgcaagtcatcatattcgcatttggagcggcaatcagaccctggaaacccgcgatcgcaaaccgttttatac
cattgatctgaatagtagtctgaccgtgccgctgcagagttgcgtgaaaccgccgtatatgctggtggttggcaatattgttattaagccggatagc
cagaccattacctgtgaaaattgtcgcctgctgacctgtattgatagcaccttaattggcagcatcgcattctgctggtcgcgcccgtgaaggcgt
ttggattccggttagtatggatcgtccgtgggaagcaagcccgagcgttcatattctgaccgaagtgctgaaaggcgtgctgaatcgtagcaaac
gttttattttaccctgattgcagtgattatgggtctgattgcagttaccgcaaccgcagcagttgccggcgttgcactgcatagtagcgttcagagcg
ttaattttgttaatgattggcagaaaaacagcacccgtctgtggaatagccagagcagtattgatcagaaactggcaaatcagattaatgatctgc
gccagaccgtttttggatgggtgaccgtctgatgagtctggaacatcgctttcagctgcagtgtgattggaataccagcgattttgtattaccccg
cagatctataatgaaagcgaacatcattgggatatggttcgccgccatctgcagggccgtgaagataatctgaccctggatattagcaaactga
aagaacagattttcgaagccagtaaagcccatctgaatctggttccgggtaccgaagccattgccggtgtggccgatggtctggccaatctgaa
tccggttacctgggtgaaaaccTAA

Amino acids sequence of the recombinant his-tagged HERV-K SU protein

SEQ ID NO:18
M*HHHHHH*LPMPAGAAAANYTYWAYVPFPPLIRAVTWMDNPTEVYVNDSVWVPGPIDDRCPAKPEEE
GMMINISIGYHYPPICLGRAPGCLMPAVQNWLVEVPTVSPICRFTYHMVSGMSLRPRVNYLQDFSYQ
RSLKFRPKGKPCPKEIPKESKNTEVLVWEECVANSAVILQNNEFGTIIDWAPRGQFYHNCSGQTQSCP
SAQVSPAVDSDLTESLDKHKHKKLQSFYPWEWGEKGISTPRPKIVSPVSGPEHPELWRLTVASHHIRI
WSGNQTLETRDRKPFYTIDLNSSLTVPLQSCVKPPYMLVVGNIVIKPDSQTITCENCRLLTCIDSTFNW
QHRILLVRAREGVWIPVSMDRPWEASPSVHILTEVLKGVLNRSKRFIFTLIAVIMGLIAVTATAAVAGVA
LHSSVQSVNFVNDWQKNSTRLWNSQSSIDQKLANQINDLRQTVIWMGDRLMSLEHRFQLQCDWNTS
DFCITPQIYNESEHHWDMVRRHLQGREDNLTLDISKLKEQIFEASKAHLNLVPGTEAIAGVADGLANLN
PVTWVKT

Production protocol:

Recombinant his-tagged soluble unit (SU) of HERV-K ENV (HERV-K6 [SU] envelope protein of 550aa; 62,3kDa; HERV-K6; GenBank accession no. DQ112120.1) was produced by GTP Bioways (Toulouse, France) according to quality control specifications of GeNeuro (Geneva, Switzerland).
Briefly following steps were performed:

- Design of pGPTc502-HERV K-6His expression vector (GTP Bioways) and transformation of E. coli bacteria BL21 T1R (DE3) strain
- Expression of the protein in 2L culture of transformed bacteria
- Monitoring of bacteria growth and harvesting

- Cells lysis
- Solubilization
- Purification on IMAC excel resin followed by gel filtration chromatography
- SDS-PAGE analysis
- Protein quantity was measured by UV assay at 280nm using Nanodrop
- Endotoxin-free conditions, Endotoxin assay and, if needed, protein polishing. Endotoxin levels for HERV-W batches were measured using Charles River Endosafe nexgen-PTS (Portable Endotoxin Testing System) test. If the level of endotoxin is >20EU/mL, an Endotoxin removal step must be performed. Endotoxin removal was done by polishing batches through Mustang Q Acrodisc followed by filtration on 0.22μm filter Stericup (Merck, Darmstadt, Germany).

Storage buffer: HERV-K ENV protein was stored in 20 mM Tris pH8, 150 mM NaCl, 1.5% SDS, 5 mM DTT, 0.5 M Urea formulation buffer.

EXAMPLE 2.2. Production of Full length HERV-K ENV (SU-TM) recombinant protein:

Sequences:

Nucleotides sequence of HERV-K SU and HERV-K TM with a signal peptide at the beginning and ended with a stop codon

SEQ ID NO:19

atgaacccatcggagatgcaaagaaaagcacctccgcggagacggagacaccgcaatcgagcaccgttgactcacaagatgaacaaaa
tggtgacgtcagaagaacagatgaagttgccatccaccaagaaggcagagccgccgacttgggcacaactaaagaagctgacgcagttag
ctacaaaatatctagagaacacaaaggtgacacaaaccccagagagtatgctgcttgcagccttgatgattgtatcaatggtggtaagtctccct
atgcctgcaggagcagctgcagctaactatacctactgggcctatgtgcctttcccgcccttaattcgggcagtcacatggatggataatcctata
gaaatatatgttaatgatagtgtatgggtacctggccccacagatgattgctgccctgccaaacctgaggaagaagggatgatgataaatatttc
cattgggtatcgttatcctcctatttgcctagggagagcaccaggatgtttaatgcctgcagtccaaaattggttggtagaagtacctactgtcagtc
ccatcagtagattcacttatcacatggtaagcgggatgtcactcaggccacgggtaaattatttacaagacttttcttatcaaagatcattaaaattt
agacctaaagggaaaccttgccccaaggaaattcccaaagaatcaaaaaatacagaagtttttagtttgggaagaatgtgtggccaatagtgc
ggtgatattacaaaacaatgaattcggaactcttatagattgggcacctcgaggtcaattctaccacaattgctcaggacaaactcagtcgtgtcc
aagtgcacaagtgagtccagctgttgatagcgacttaacagaaagtttagacaaacataagcataaaaaattgcagtctttctacccttgggaat
ggggagaaaaaggaatctctaccgcaagaccaaaaataataagtcctgtttctggtcctgaacatccagaattatggaggcttactgtggcctc
acaccacattagaatttggtctggaaatcaaactttagaaacaagagatcgtaagccattttatactatcgacctaaattccagtctaacagttcctt
tacaaagttgcgtaaagcccccttatatgctagttgtaggaaatatagttattaaaccagactcccagactataacctgtgaaaattgtagattgctt
acttgcattgattcaactttttaattggcaacaccgtattctgctggtgagagcaagagagggcgtgtggatTcctgtgtccatggaccgaccgtgg
gaggcctcaccatccgtccatattttttgactgaagtattaaaaggtgtttttaaatagatccaaaagattcatttttactttaattgcagtgattatgggatt
aattgcagtcacagctacggctgctgtagcaggagttgcattgcactcttctgttcagtcagtaaactttgttaatgattggcaaaataattctacaa
gattgtggaattcacaatctagtattgatcaaaaattggcaaatcaaattaatgatcttagacaaactgtcatttggatgggagacaggctcatga
gcttagaacatcgtttccagttacaatgtgactggaatacgtcagattttttgtattacaccccaaatttataatgagtctgagcatcactgggacatg
gttagatgccatctacagggaagagaagataatctcactttagacatttccaaattaaaagaacaaattttttgaagcatcaaaagcccatttaaat
ttggtgccaggaactgaggcaattgcaggagttgctgatggcctcgcaaatcttaacactgtcacttgggttaagacc**attggaagtactacaa
ttataaatctcatattaatccttgtgtgcctgtttttgtctgttgttagtctacaggtgtacccaacagctccgacgagacagcgaccatcg**

**agaacgggccatgatgacgatggtggtttttgtcgaaaagaaaaggggggaaatgtggggaaaagcaagagagatcagattgttact
gtgtctgtg**TAG

Amino acids sequence of the recombinant full length HERV-K ENV (SU-TM) protein with a signal peptide at the beginning

SEQ ID NO:20

MNPSEMQRKAPPRRRRHRNRAPLTHKMNKMVTSEEQMKLPSTKKAEPPTWAQLKKLTQLATKYLEN
TKVTQTPESMLLAALMIVSMVVSLPMPAGAAAANYTYWAYVPFPPLIRAVTWMDNPIEIYVNDSVWVP
GPTDDCCPAKPEEEGMMINISIGYRYPPICLGRAPGCLMPAVQNWLVEVPTVSPISRFTYHMVSGMSL
RPRVNYLQDFSYQRSLKFRPKGKPCPKEIPKESKNTEVLVWEECVANSAVILQNNEFGTLIDWAPRGQ
FYHNCSGQTQSCPSAQVSPAVDSDLTESLDKHKHKKLQSFYPWEWGEKGISTARPKIISPVSGPEHPE
LWRLTVASHHIRIWSGNQTLETRDRKPFYTIDLNSSLTVPLQSCVKPPYMLVVGNIVIKPDSQTITCENC
RLLTCIDSTFNWQHRILLVRAREGVWIPVSMDRPWEASPSVHILTEVLKGVLNRSKRFIFTLIAVIMGLIA
VTATAAVAGVALHSSVQSVNFVNDWQNNSTRLWNSQSSIDQKLANQINDLRQTVIWMGDRLMSLEHR
FQLQCDWNTSDFCITPQIYNESEHHWDMVRCHLQGREDNLTLDISKLKEQIFEASKAHLNLVPGTEAIA
GVADGLANLNTVTWVKT**IGSTTIINLILILVCLFCLLLVYRCTQQLRRDSDHRERAMMTMVVLSKRKGG
NVGKSKRDQIVTVSV**

Production protocol

Full length recombinant HERV-K ENV (full-length HERV-K113 envelope protein [SU-TM] of 699aa; 79,2kDa; HERV-K113; GenBank accession no. NC_022518.1) was produced by Geneuro Innovation (Lyon, France) according to quality control specifications of GeNeuro (Geneva, Switzerland).

Briefly following steps were performed:

- The nucleotide sequence encoding full length HERV-W ENV (SU-TM) was clone in phCMV-LTR expression vector
- HEK-293T cells were transfected with phCMV-ENV-LTR-K113 using Lipofectamine 2000 transfection kit (Invitrogen 11668-019) in OptiMEM medium (Invitrogen 31985-047).
- Cells were harvested 48 hours post transfection
- Cells were lysed 2h at room temperature with gentle agitation in 200mM Tris pH8, 150mM NaCl, 6M urea,0.6% NP40, 2mM EDTA, 1mM PMSF, 1X "Complete" protein inhibitor
- Lysis product was centrifuged 10 min at 10 000g (9 500 rpm) at 4°C
- Mix of extracted proteins containing HERV-K ENV (SU-TM) were immediately stored at -80°C

EXAMPLE 2.3. Production of Frameshifted HERV-K ALT (SU-ALT) recombinant protein

In order to reproduce the consequences of the frameshifted translation of the regular HERV-K *ENV* gene sequence, a modified sequence has been inserted into an expression plasmid to readily express the C-term alternative protein (SU-ALT).

This allows to produce a protein HERV-K ENV with an alternative C-term ending highly hydrophobic. Thanks to this hydrophobic ending, HERV-K ENV recombinant protein monomers are capable of forming oligomers by incubation in a solubilization buffer. This makes it possible to produce a calibration curve of purified recombinant antigen equivalent to the native antigen isolated from the serum of patients with pathologies linked to HERV-K ENV expression. This makes it possible to produce a tool which was lacking and which is essential for carrying out the quantitative diagnostic test of the present application.

*EXAMPLE 2.3.1: Purified recombinant protein produced in procaryote system:*

Sequences:

Nucleotides sequence of HERV-K SU preceded by a signal peptide and followed by Ribosomal frameshift locus, a **frameshifted ALT sequence,** a *Linker Sequence,* a *His-tag sequence and a stop codon*

SEQ ID NO:21

catatgaatccgtctgaaatgcaacgtaaagctccgccacgtcgtcgtcgtcaccgtaatcgtgctccgctgacccacaaaatgaataaaatgg
ttacctctgaagaacagatgaaactgccgtctaccaaaaaagcggaaccgccgacctgggcgcagctgaagaaactgactcagctggctac
caaatatctggaaaatactaaagtaacccagactccagaatccatgctgctggctgcactgatgattgtttccatggtagtttccctgccgatgcca
gctggtgcggcagctgccaattacacctactgggcatacgtgccgttcccgccgctgattcgtgcagtgacctggatggataatccgattgaaat
ctatgtaaatgattctgtttgggtaccgggtccgactgatgactgctgcccggccaaaccggaagaagaaggcatgatgattaatatcagcatcg
gttaccgctatccgccaatctgcctgggtcgtgcaccgggttgcctgatgccggcggttcagaattggctggttgaagtgccgaccgtaagcccg
atttctcgttttacctaccacatggtttctggcatgtccctgcgtccgcgtgtgaattacctgcaagacttcagctaccagcgttctctgaaattccgcc
cgaagggcaaaccgtgtccgaaggaaatcccgaaggaaagcaagaataccgaagtgctggtttgggaagagtgtgtagcgaatagcgctg
tgatcctgcagaataatgagtttggtaccctgattgactgggctccgcgtggtcagttctaccacaattgcagcggtcagacccagtcttgtccgtct
gcacaggtttctccggcggttgattctgacctgaccgaaagcctggacaaacacaaacacaagaaactgcagtctttctatccgtgggaatgg
ggcgaaaaaggcatcagcactgcccgcccaaaaatcatctctccagttagcggtccggagcatccagagctgtggcgtctgactgttgcctctc
accacatccgcatctggtccggtaatcagactctggagactcgtgaccgcaaaccattctacaccattgacctgaattccagcctgactgttccg
ctgcagtcttgcgtgaaaccgccgtacatgctggtagtaggcaatatcgtaatcaaaccagattcccagaccatcacctgcgagaattgccgcc
tgctgacttgcattgactccaccttcaattggcagcaccgcatcctgctggtgcgtgcgcgcgaaggcgtgtggattccggtttccatggatcgtcc
gtgggaagcgtctccgtctgtgcacatcctgactgaagtgctgaaaggcgttctgaat<ins>cgctccaaacgc</ins>**caaaaaatccattttttacttcaatt**
**gttccgactatggcatcaattgcagccacagctacggttgttgttcccgttcctgcatcgcgctgttctgcagcgtttccaaactgtgt**<ins>g</ins>
<ins>gttccagc</ins>catcatcaccaccaccatTAATAA

Production protocol:

Recombinant his-tagged HERV-K ALT (HERV-K SU-ALT protein of 512aa; 58,2kDa; custom sequence from HERV-K *ENV* DNA prediction of frameshifted expression) was produced by GTP Bioways (Toulouse, France) according to quality control specifications of GeNeuro (Geneva, Switzerland).

Briefly following steps were performed:

- Design of pGPTc502HERV-K_ALT expression vector (GTP Bioways) and transformation of E. coli bacteria BL21 T1R (DE3) strain
- Expression of the protein in 3L culture of transformed bacteria
- Monitoring of bacteria growth and harvesting
- Cells lysis
- Solubilization
- Purification on IMAC excel resin followed by gel filtration chromatography
- SDS-PAGE analysis
- Protein quantity was measured by UV assay at 280nm using Nanodrop
- Endotoxin-free conditions, Endotoxin assay and, if needed, protein polishing

Endotoxin levels for HERV-K ALT batches were measured using Charles River Endosafe nexgen-PTS (Portable Endotoxin Testing System) test. If the level of endotoxin is >20EU/mL, an Endotoxin removal step must be performed.

Endotoxin removal was done by polishing batches through Mustang Q Acrodisc followed by filtration on 0.22$\mu$m filter Stericup (Merck, Darmstadt, Germany).

Storage buffer: HERV-K ALT (SU-ALT) protein was stored in 20 mM Tris, 150 mM NaCl, 1.5% SDS, 5 mM b-mercaptoethanol, 0.5 M Arginine formulation buffer.

*EXAMPLE 2.3.2: Non purified recombinant protein produced in eucaryotic system:*

Sequences:

Nucleotides sequence of HERV-K SU preceded by a signal peptide and followed by <ins>Ribosomal frameshift locus, a</ins> **frameshifted ALT sequence,** a *<ins>Linker</ins> Sequence* , a *His-tag sequence and a stop codon*

SEQ ID NO:21

catatgaatccgtctgaaatgcaacgtaaagctccgccacgtcgtcgtcgtcaccgtaatcgtgctccgctgacccacaaatgaataaaatgg
ttacctctgaagaacagatgaaactgccgtctaccaaaaaagcggaaccgccgacctgggcgcagctgaagaaactgactcagctggctac
caaatatctggaaaatactaaagtaacccagactccagaatccatgctgctggctgcactgatgattgttccatggtagtttccctgccgatgcca
gctggtgcggcagctgccaattacacctactgggcatacgtgccgttcccgccgctgattcgtgcagtgacctggatggataatccgattgaaat
ctatgtaaatgattctgtttgggtaccgggtccgactgatgactgctgcccggccaaaccggaagaagaaggcatgatgattaatatcagcatcg
gttaccgctatccgccaatctgcctgggtcgtgcaccgggttgcctgatgccggcggttcagaattggctggttgaagtgccgaccgtaagcccg
atttctcgtttttacctaccac**atggtttctggcatgtccctgcgtccgcgtgtgaattacctgcaagacttcagctaccagcgttctctgaaattccgcc
cgaagggcaaaccgtgtccgaaggaaatcccgaaggaaagcaagaataccgaagtgctggtttgggaagagtgtgtagcgaatagcgctg
tgatcctgcagaataatgagtttggtaccctgattgactgggctccgcgtggtcagttctaccacaattgcagcggtcagacccagtcttgtccgtct
gcacaggtttctccggcggttgattctgacctgaccgaaagcctggacaaacacaaacacaagaaactgcagtctttctatccgtgggaatgg
ggcgaaaaaggcatcagcactgcccgcccaaaaatcatctctccagttagcggtccggagcatccagagctgtggcgtctgactgttgcctctc
accacatccgcatctggtccggtaatcagactctggagactcgtgaccgcaaaccattctacaccattgacctgaattccagcctgactgttccg
ctgcagtcttgcgtgaaaccgccgtacatgctggtagtaggcaatatcgtaatcaaaccagattcccagaccatcacctgcgagaattgccgcc
tgctgacttgcattgactccaccttcaattggcagcaccgcatcctgctggtgcgtgcgcgcgaaggcgtgtggattccggttccatggatcgtcc
gtgggaagcgtctccgtctgtgcacatcctgactgaagtgctgaaaggcgttctgaat**<u>cgctccaaacgc</u>**caaaaaatccatttttacttcaatt
gttccgactatggcatcaattgcagccacagctacggttgttgttcccgttcctgcatcgcgctgttctgcagcgtttccaaactgtgt**<u>g
gttccagc</u>*catcatcaccaccaccat*TAATAA

Amino acid sequence of a recombinant HERV-K ALT protein preceded by and followed by <u>Ribosomal frameshift locus,</u>
<u>a</u> **frameshifted ALT sequence,** a *Linker Sequence,* a *His-tag sequence and a stop codon*

SEQ ID NO:22

MNPSEMQRKAPPRRRRHRNRAPLTHKMNKMVTSEEQMKLPSTKKAEPPTWAQLKKLTQLATKYLEN
TKVTQTPESMLLAALMIVSMVVSLPMPAGAAAANYTYWAYVPFPPLIRAVTWMDNPIEIYVNDSVWVP
GPTDDCCPAKPEEEGMMINISIGYRYPPICLGRAPGCLMPAVQNWLVEVPTVSPISRFTYHMVSGMSL
RPRVNYLQDFSYQRSLKFRPKGKPCPKEIPKESKNTEVLVWEECVANSAVILQNNEFGTLIDWAPRG
QFYHNCSGQTQSCPSAQVSPAVDSDLTESLDKHKHKKLQSFYPWEWGEKGISTARPKIISPVSGPEH
PELWRLTVASHHIRIWSGNQTLETRDRKPFYTIDLNSSLTVPLQSCVKPPYMLVVGNIVIKPDSQTITCE
NCRLLTCIDSTFNWQHRILLVRAREGVWIPVSMDRPWEASPSVHILTEVLKGVLN<u>RSKR</u>**QKIHFYFNC
SDYGINCSHSYGCCSRSCIALFCSVSKLC**<u>*GSS*</u>*HHHHHH*

<u>Production protocol</u>

Recombinant his-tagged HERV-KALT (HERV-K SU-ALT protein of 512aa; 58,2kDa); original sequence was produced by GeNeuro Innovation (Lyon, France) according to quality control specifications of GeNeuro (Geneva, Switzerland).
Briefly following steps were performed:

- The nucleotide sequence encoding full length HERV-K ALT (SU-ALT) was clone in phCMV-LTR expression vector
- HEK-293T cells were transfected with phCMV-EnvK113-SU-ALT using Lipofectamine 2000 transfection kit (Invitrogen 11668-019) in OptiMEM medium (Invitrogen 31985-047).
- Cells were harvested 48 hours post transfection
- Cells were lysed 2h at room temperature with gentle agitation in 200mM Tris pH8, 150mM NaCl, 6M urea,0.6% NP40, 2mM EDTA, 1mM PMSF, 1X "Complete" protein inhibitor
- Lysis product was centrifuged 10 min at 10 000g (9 500 rpm) at 4°C
- Mix of extracted proteins containing HERV-K ALT (SU-ALT) were immediately stored at -80°C

**EXAMPLE 3: In vitro production of oligomers from purified recombinant proteins of HERV envelopes or HERV envelopes with alternative reading frames.**

**Materials and Methods**

**Table 2: Biological and chemical reagents and materials**

| Material or Reagent name | Reagent type | Reference | Supplier |
|---|---|---|---|
| recombinant HERV-W ENV | Recombinant protein produced in E. coli | n/a | GTP Bioways |
| GN_mAb_Env01 | Murine monoclonal antibody | n/a | GNI |
| Sample Buffer 10X | Denaturing buffer for protein dilution | 042-195 | Bio-Techne |
| Fos Choline 16 | Detergent well suited for the solubilization, stabilisation, and purification of membrane proteins | F316S-1GM | Anatrace |
| Sodium Dodecyl Sulfate (SDS) | Reductive detergent | 74255 | Sigma |
| Foetal Calf Serum (FCS) | Decomplemented bovin serum | 30-2022 | ATCC |
| 1X DMEM/F12 | Cell culture medium | 31331-028 | Gibco |
| Lipofectamin 2000 | Transfection reagent | 11668-019 | Invitrogen |
| Mem-PER Plus Membrane Protein Extraction Kit | Protein Purification and Isolation reagents | 89842 | ThermoFisher Scientific |

**Table 3: Simple Western materials and reagents**

| Material or Reagent name | Reference | Supplier |
|---|---|---|
| Simple Western Automated Western Blot instrument | n/a | ProteinSimple |
| 66-440 kDa Separation Module | SM-WO08 | ProteinSimple |
| Secondary Streptavidin HRP | 043-459-2 | ProteinSimple |
| Anti-Mouse Secondary HRP Antibody | 042-205 | ProteinSimple |
| Antibody Diluent 2 | 042-203 | ProteinSimple |
| Peroxide | 043-379 | ProteinSimple |
| Luminol-S | 043-311 | ProteinSimple |
| Sample Buffer 10X | 042-195 | ProteinSimple |

Protein preparation:

As presented in **Figure 2,** HEK293T cells were cultured at 37 °C and 5% CO2 in DMEM/F-12 (Gibco, 31331-028) supplemented with 10% of heat-inactivated (30 min at 56 _C) bovine FBS (ATCC, 30-2020) and 10 $\mu$L Penicillin-Streptomycin/mL (Sigma, P4333). 250,000 HEK cells were transfected with 3 $\mu$g of plasmid using Lipofectamine 2000 transfection reagent (Invitrogen, 11668-019). The following plasmid constructions under the control of CMV promoter (GeNeuro, Switzerland) were transfected: pMAX-GFP, encoding GFP and used as control, pCMV-HERV-W ENV encompassing the complete ORF of HERV-W ENV (cDNA clone from MS cell culture virion RNA encoding 542 amino acids GenBank no. AF331500.1), pCMV-HERV-K ENV encompassing the complete ORF of ENV from HERVK113 clone (Beimforde et al. 2008; Hanke et al. 2009); pCMV-HERV-K ALT encompassing the complete ORF of ENV -SU from HERV-K113 clone followed by the additional sequence encoding for ALT polypeptide. Transfected cells were harvested 24-48 h post transfection. The ALT polypeptide was synthesized by Smart Bioscience, Saint Egrève, France.

As presented in **Figure 3,** the purified recombinant full-length HERV-W ENV protein was produced in *E. coli* and solubilised in buffer containing 20 mM Tris pH8, 150 mM NaCl, 1.5% SDS, 5 mM DTT, 0.5 M Urea (GTP Bioways). Recombinant HERV-W ENV protein was incubated 24 h at 37°C in 1X DMEM/F12 (Gibco, 31,331-028) completed with a combination of several reagents: 1.5% SDS (Sigma, 74,255-250G); 10% fetal calf serum (FCS) (ATCC, 30-2022); 10% BSA (Sigma; A7906) and 1% fos choline 16 (Anatrace, F316S-1GM). For the presented immunocapillary analyses, 1$\mu$g of HERV-W ENV was incubated for 10 min at room temperature (RT) with 50$\mu$L of Sample buffer (ProteinSimple)

Simple Western analysis:

Protein solutions were analyzed on Simple Western technology device (Wes, Jess, Leo, Abby), an automated capillary-based size sorting and immunolabeling system (ProteinSimpleTM). All protocols were performed with manufacturer's reagents according to their manual. Briefly, prepared CSFs were mixed with fluorescent master mix and heated at 95 °C for 5 min. The samples, blocking reagent, wash buffer, 20 μg/mL of primary antibody: humanized temelimab (T1940422-A, GeNeuro) and its ready to use detection module (DM-005, ProteinSimple: anti-human HRP-coupled secondary antibody), and chemiluminescent substrate were dispensed into the microplate. Samples were loaded in triplicates into individual capillaries on a 25 capillary cartridge (66-440 kDa Jess separation module, SM-WO08, ProteinSimple). Protein separation and immunodetection was performed automatically on individual capillaries using default settings. The global signal (AUC) containing the target hexamer HERV-W ENV (electrophoregram peak about 400 kDa) was measured using Compass software for Simple Western devices settings and raw data recording (ProteinSimple/Biotechne).

EXAMPLE 3.1: A highly hydrophobic extremity is required for HERV protein auto-assembly: evidence form HERV-K ENV versus HERV-K ALT, respectively without and with a highly hydrophobic extremity

HERV envelope proteins presenting either a strongly hydrophobic transmembrane domain in one extremity or expressing alternative forms of HERV protein (SU-ALT) generating highly hydrophobic extremity caused by ribosomal frameshifting or other transcription/translation regulation mechanism producing a protein with a hydrophobic extremity, oligomerized when these proteins were obtained by cellular transfection in appropriate cell-culture medium (**Figure 2**).
Indeed, HERV elements, such as HERV-W ENV (**Figure 2 A-A'**), possess a highly hydrophobic domain and produce several antigenic forms including a high molecular weight oligomer, corresponding to an hexamer as can be expected from its molecular weight with little variation due to few possible glycosylations on this protein. HERV-K ENV which does not possess hydrophobic domain at the extremity, is not able to form any oligomer (**Figure 2 B, B'**). The frameshifted form of HERV-K ENV, HERV-K ALT, presenting the replacement of the regular extremity of HERV-K ENV with low hydrophobicity, by the highly hydrophobic alternative (ALT) polypeptide (**Figure 2 C, C'**) at its C-terminal extremity, showed the appearance of oligomer formation (**Figure 2 D, D'**).
This allows to produce a protein HERV-K ENV with an alternative C-term ending highly hydrophobic. Thanks to this hydrophobic ending, HERV-K ENV recombinant protein monomers are capable of forming oligomers by incubation in a solubilization buffer. This makes it possible to produce a calibration curve of purified recombinant antigen equivalent to the native antigen isolated from the serum of patients with pathologies linked to HERV-K ENV expression. This makes it possible to produce a tool which was lacking and which is essential for carrying out the quantitative diagnostic test of the present application.

EXAMPLE 3.2: Monomeric HERV-W ENV and HERV-K ENV/ALT recombinant proteins can be readily oligomerized in the presence of Mem-PER solubilization buffer

Oligomerization ability of HERVs proteins possessing highly hydrophobic domain at one extremity is dependent on the reaction medium. Oligomerization experiments had always failed with all chemical media tested so far. The few biological culture media identified for transfection-produced proteins allowed oligomerization but could not be used for large-scale oligomerized protein production and purification. Moreover, they did not fit with the needs and quality for developing recombinant standards in calibration curves of research-use only or diagnostic dosage tests.
Purified recombinant HERV-W ENV, which is a HERV envelope presenting a hydrophobic extremity, showed an isoelectric pH (pHi) above 9, implying electropositivity of its polypeptide structure at physiological (neutral) pH.
Of note, this induces electrostatic interaction with plastic surfaces and a rapid loss of recombinant protein when diluted in media such as PBS1X (data not shown) or DMEM 1X (**Figure 3 A-C**). Kinetics of disappearance of the protein from the aqueous medium revealed a 50% loss after 10min of incubation at 37°C in DMEM and undetectable protein in the solution after 3h (**Figure 3 C**). This electrostatic capture of HERV-W ENV was prevented with 10% fetal calf serum (FCS), which blocks plastic charges with serum proteins but may also offer alternative electrostatic bonds in solution for HERV-W ENV at neutral pH (**Figure 3 D-F**). This is not favorable for obtaining a purified oligomerized protein and represents another challenge for separating these biological molecules.
It has been also analyzed the immunodetection profile of the non-glycosylated *E. coli*-recombinant protein diluted and incubated in medium containing 10% fetal calf serum by capillary electrophoresis (WES, Bio-Techne/ProteinSimple, USA) under denaturing conditions and nonetheless evidenced that HERV-W ENV is detected under monomeric and oligomeric forms: a monomer (62 kDa) and, a high molecular weight (MW) oligomer (380-440 kDa) (**Figure 3 D-F**). Repeated analyses in the same conditions confirmed that HERV-W ENV partly yields an hexameric structure, fitting with the MW of six monomers within the limits of resolution of the separation matrix in capillaries. Kinetics studies with recombinant monomers in 10% FCS with cell culture medium (DMEM 1X) evidenced a dynamic process with a relative decrease in monomers over time stabilizing after a parallel increase of hexamers representing the dominant form (**Figure 3 F**). Further study of electrostatic forces responsible of unbound HERV-W ENV disappearance in protein-free aqueous media was performed after this oligomeric profile was characterized. 1.5% SDS was added in DMEM medium to neutralize the electropositivity of HERV-W ENV and, after overnight incubation at 37°C, the monomer was still present in the medium, but no hexamer was obtained. This showed that oligomerization of monomers can be inhibited by SDS, which is used in most buffers for protein extraction and denaturation (**Figure 3 G, H**).

Previous observations had also indicated a loss of HERV-W ENV detection when spiked in total serum. It has been therefore addressed a possible interaction with albumin which represents 50% of serum proteins. Beyond its global negative charge like other plasma proteins at physiological pH, albumin efficiently binds hydrophobic moieties of various molecules. Sufficient addition of bovine serum albumin (BSA) to DMEM with 10% FCS inhibited the detection of HERV-W ENV in either monomeric or hexameric forms. Results supported a masking role of albumin above a concentration threshold not reached when using 10% FCS in the medium. This is likely to involve the hydrophobic moiety of HERV-W ENV protein in hydrophobic interactions with serum albumin (Fatima et al., 2017a, b) and indicates that this creates a major obstacle for analyzing and dosing this protein from total serum of human patients with various conditions. Hypothesizing effective hydrophobic interactions with albumin in solution, total proteins have been extracted from this mixture with BSA using 1% fos choline 16 diluted in RIPA buffer to disrupt macromolecular complexes formed by both electrostatic and hydrophobic bonds. In the presence of fos choline 16, and not without, it could rescue, but only partially, HERV-W ENV detection (**Figure 3 G, H**). This indicated that fos choline 16 in RIPA buffer could be used for an extraction of HERV-W ENV antigens in the global analyses of cultured cells but cannot allow a reliable dosage of the actual protein present in a biological sample with such a partial and variable percentage of extracted hexamer.

While these conditions were suitable for research analyses and interpretation of physico-chemical properties of these envelope proteins, they failed to match requested conditions for developing a robust detection with an absolute quantification for further research or diagnostic use in real human body fluids or tissues.

It is now described that unexpected results have been observed during experiments using one component of the Mem-PER protein extraction kit, under conditions that differed from the recommended use of this kit, among the various chemical compounds of buffers tested with this protein: when purified HERV-W ENV recombinant protein was diluted in the "solubilization buffer" from the "Mem-PER kit" and incubated for a few minutes at room temperature, i.e. from 20 to 40 min such as 30 min, a fully oligomerized HERV-W ENV has been obtained, which corresponds to the antigenic form found in human biological fluids without significant remains of the incubated monomer. (**Figure 4**).

## EXAMPLE 4: Production of anti-HERV-W ENV antibodies

EXAMPLE 4.1: Immunization protocol and generation of hybridomas

The anti-MSRV/HERV-W ENV antibodies were produced according to the protocols described below:
Mice were immunized with recombinant ENV protein expressed from cloned RT-PCR regions (Full-length protein, Surface-SU- fragment, Transmembrane fragment and SU-TM junction region) amplified from purified extracellular MSRV virions (MRSV/ENV protein). Briefly, recombinant MSRV proteins tested were produced in E. coli from clone pV14 (env/AF331500), corresponding to cDNA clones amplified as described (Komurian-Pradel, 1999; Perron, 2001). His-tagged proteins were produced and purified by Amplicon Express (WA) and controlled by SDS$\pm$PAGE and Western blot analyses.

BALB/c mice were immunized according to the following protocol: On day 0, mice received an intraperitoneal (i.p.) injection of 20 $\mu$g of the purified recombinant MSRV (HERV-W) ENV protein in the presence of complete Freund's adjuvant. On day 14 and day 28, mice received a further i.p. injection of the same amount of MSRV/ENV protein in the presence of incomplete Freund's adjuvant. Four, three and two days before fusion, mice received three additional i.p. booster doses of 100 $\mu$g of MSRV/ENV protein diluted in physiological saline.

Hybridomas were generated by fusing mouse spleen cells with the non-Ig-secreting, 8-azaguanine-resistant myeloma cell line Sp2/0-Ag14. Hybridomas were grown in Iscove's modified Dulbecco's medium (IMDM; Gibco BRL 21980-032) with 25 mM HEPES and L-glutamine and supplemented with 50 mM $\beta$-2-mercaptoethanol (Invitrogen 31350-010), 20 mM ethanol amine (Sigma E0135), 4 mg/ml insulin (Sigma J9278), 10% heat inactivated fetal calf serum and antibiotics (20'000U/ml Penicillin, 20'000 $\mu$g/ml Streptomycin). Hybrids were selected in HAT containing IMDM medium by plating in microtiter plates. Cell culture supernatants from resulting hybridomas were assayed for anti-MSRV/HERV-W ENV antibodies in ELISA using coated recombinant HERV-W/MSRV proteins of interest, and if positive, were cloned by limiting dilution.

For screening and analysis of antibodies in cell culture supernatants the following ELISA was performed. Briefly, ELISA plates were coated with 100$\mu$l of recombinant ENV protein at 1$\mu$g/ml in 0.05 M bicarbonate buffer, pH 9.6. The coated plates were incubated overnight at 18-22°C. The plates were then blocked by incubation with 200 $\mu$l of PBS containing 1% milk for 1 hour at 37°C. 100 $\mu$l of culture supernatants diluted in PBS containing 0.05% Tween 20 were added and the plates were incubated for 1 hour at 37°. After washing the plates three times with PBS-0.05% Tween 20, 100 $\mu$l of goat anti-mouse Ig (H+L) polyclonal antibody conjugated to alkaline phosphatase (Jackson Immunoresearch ref:

115-055-062), diluted in PBS-1% BSA, to 1/2000, were added and the plates were incubated for 1 hour at 37°. Antibody-binding was visualized by adding 100 µl of PNPP (Biomérieux ref 60002990) at a concentration of 2 mg/ml in DEA-HCL (Biomérieux ref 60002989), pH=9.8, and incubation for 30 minutes at 37°C. Color reaction was stopped with 1N NaOH. Optical density was read on an ELISA-plate reader connected to dedicated data registration and analysis software (bioMérieux, France).

*Multiple fusions were performed*

a) From the screening with a protein encompassing the **"ENV SU" ectodomain** a total of 400 supernatants were screened by the ELISA technique. After initial screening, 22 supernatants were found to be positive by ELISA with an OD >0.2, corresponding to four times the background noise. After further propagation, a single hybridoma secreting high affinity anti-MSRV/HERV-W ENV antibody was identified (antibody Env02).

b) From the screening with a protein encompassing the **complete protein with "SU TM" domains.**

the following modification in the immunization protocol were performed: Instead of 20 µg, mice were immunized i.p. on day 0 with 40 µg of the purified recombinant MSRV/ENV protein in complete Freund's adjuvant, followed by i.p. immunizations on days 14, 28 and 78, with the same dose in the presence of incomplete Freund's adjuvant. The three booster doses before fusions were given i.p. with 50 µg per dose (instead of 100 µg per dose) in physiological saline. After fusion, 1350 supernatants were screened by the indirect ELISA technique, as described above. A total of 39 supernatants were found to be positive by ELISA with an OD >0.4, corresponding to four times the background noise. After further propagation, among other isolated antibodies, the antibody GN_mAb_Env01 was selected.

The antibody GN_mAb_Env01, also called GNbAC1, comprises each of the complementary-determining regions (CDRs) set forth in SEQ ID NO: 1, SEQ ID NO: 2, SEQ ID NO: 3 SEQ ID NO: 4, SEQ ID NO: 5 and SEQ ID NO: 6.

More particularly, it comprises:

- a light chain comprising a light chain variable region (VL) which comprises three complementarity determining regions: CDR-L1 as set forth in SEQ ID NO:1, CDR-L2 as set forth in SEQ ID NO:2 and CDR-L3 as set forth in SEQ ID NO:3,

- a heavy chain comprising a heavy chain variable region (VH) which comprises three complementarity determining regions: CDR-H1 as set forth in SEQ ID NO:4, CDR-H2 as set forth in SEQ ID NO:5 and CDR-H3 as set forth in SEQ ID NO:6.

EXAMPLE 4.2: Production and purification of MAb

Selected hybridomas were either adapted as ascites tumors in pristine-primed BALB/c mice or were grown in tissue cultures. Irrespective of the method of production, as supernatant or as ascites, the antibodies are then purified. The purification methods used are essentially filtration on ion exchange gel and exclusion chromatography or affinity chromatography (protein A or G). The antibody concentration of the purified samples was determined by OD 280nm t isolated peak of elution by FPLC and the purity was >99% as determined by final control HPLC elution profile analysis.

As described below, all the following experiments were performed with purified antibody preparations. The antibodies were screened in functional assays to select the most effective antibodies.

**EXAMPLE 5: Example of methodological procedure for the characterization of anti-HERV-W ENV antibodies**

To characterize GN_mAb_Env01 antibody, several technologies were used: denaturing/reducing Simple Western, native ELISA and immunofluorescence (IF) or immunohistochemistry (IHC) on paraformaldehyde fixed tissues.

Different antigen sources were used depending on the technology used:

- Simple Western and ELISA: Purified HERV-W ENV protein produced in procaryote system (without any post-translational modification)
- Simple Western: Lysate of HEK293T cells transfected with expression vector encoding GFP (negative control), Syncytine-1 (86% of homology with HERV-W ENV, used as specificity control) or HERV-W ENV. Protein extraction was performed using 1% Fos Choline16 in RIPA buffer.

- IF: Culture of HEK293T cells transfected with expression vector encoding GFP (negative control), Syncytine-1 (86% of homology with HERV-W ENV, used as specificity control) or HERV-W ENV.
- IHC: Paraffin embedded human tissues from COVID-19 necropsies.

Antibodies characterizations were performed on Simple Western platform (as described in EXAMPLE 3). Purified recombinant protein or transfected cells lysate were used as "template" and each anti-HERV-W ENV antibody was used as detection antibody.

EXAMPLE 5.1: Simple Western profiles obtained with GN mAb Env01 antibody.

The GN_mAb_Env01 antibody was characterized on the Simple Western platform using purified recombinant HERV-W ENV protein produced in a bacterial system (without post-translational modification) and on lysates of cells transfected (see preparation in EXAMPLE 3) with plasmids encoding GFP (negative control), syncytin-1 (specificity control) and HERV-W ENV (positive control).

As can be seen in Figure 5 A, which shows an electrophoregram of the immunodetection of purified recombinant protein produced in E. coli and detected with the GN_mAb_Env01 antibody, a dominant peak migrating at ~60kDA was observed. As expected, this peak corresponds to the MW of HERV-W ENV.

Electrophoresis of HERV-W ENV protein immunodetection in lysates of cells transfected with GFP, Syncytin-1 and HERV-W ENV (Figure 5 B) shows, notably on the digital Western Blot representation, that the GN_mAb_Env01 antibody is highly specific for HERV-W ENV. No signal is observed in lysates of cells transfected with GFP and Syncytin-1. In lysates of HERV-W ENV transfected cells, two peaks have been observed. A peak at 116 kDa which corresponds to the monomeric HERV-W ENV protein having received post-translational modifications, mainly glycosylations, and a form at ~400-440 kDa which corresponds to the hexameric form of HERV-W ENV, which is found in the biological fluids of human patients with certain diseases, e.g., multiple sclerosis (MS).

EXAMPLE 5.2: ELISA profiles obtained with GN mAb Env01 antibody.

**Table 4: List of material and reagent**

| Products | Supplier | Reference | Stockage |
|---|---|---|---|
| 10 X PBS buffer pH 7,4 | Gibco | 70011-044 | RT or +4°C |
| Carbonate-bicarbonate | Sigma | C3041-100CAP | RT |
| Tween 20 | Sigma | P7949-500ML | RT |
| BSA | Sigma | A7906-500G | +4°C |
| TMB substrate | Sigma | T0440-1L | +4°C |
| H2SO4 (0.5M) | Merck Millipore | 1.09072.1000 | RT |
| H2O sterile purified water | Otec | 600504 | RT |
| HERV-W-ENV protein | GTP | 1518-HERV-13p | -80°C |
| GN_mAb_ENV01 | Biotem | vt180514c-4913 | -20°C |
| Peroxidase-conjugated AffiniPure Goat anti-mouse IgG (H+L) | Jackson IR | 115-035-146 | +4°C |

| Material | Supplier | Reference |
|---|---|---|
| 96-well plate, POLYSORP | NUNC/thermoFischer | 475094 |
| Glomax Explorer® Reader | Promega | GM3500 |

• Coating of a range of HERV-W-ENV protein:

Important note: Do not use the vortex during the HERV-W ENV range preparation, mix the preparation only by pipetting up and down.

The POLYSORP plate were coated with 0.5 μg/mL of recombinant HERV-W ENV. 100 μL of protein were distributed in each well and incubated overnight at +4°C.

• Blocking:

Wells were washed 4 times with 200 μL/well with washing buffer. After each wash, wait for 1 minute before inverting the plate on absorbent paper to remove any residual buffer. 200 μL/well of the blocking solution were added and the plate was sealed and incubated for 1 hour at RT.

• Incubation with antibody:

Preparation of GN_mAb_Env01 dilution range by successive dilutions in the blocking buffer:
Range: 1μg/mL - 500 ng/mL - 250 ng/mL - 125 ng/mL - 62.5 ng/mL - 31.25 ng/mL - 15.63 ng/mL-7.81 ng/mL - 3.9 ng/mL - 1.95 ng/mL - 0.97 ng/mL - 0.1953 ng/mL - 0.0976 ng/mL - 0.0488 ng/mL-0.0244 ng/mL-0.00012 ng/mL -0.00006.1 ng/mL-0 ng/mL.
The condition with "0 ng/mL" corresponds to the coating buffer alone.
100 μL of each antibody concentration were distributed per well. The plate was sealed and incubated for 2 hours at 37°C.
Wells were washed 4 times with 200 μL/well of the washing buffer. After each wash, wait for 1 minute before inverting the plate on absorbent paper to remove any residual buffer. 200 μL/well of the blocking solution were added and the plate was sealed and incubated for 1 hour at RT. 50 ng/mL GN_mAb_Env01 was prepared in the blocking buffer and spread 100 μL/well. The plate was sealed and incubated for 1 hour at RT.

• Incubation with the detection antibody:

Wells were washed 4 times with 200 μL/well of the washing buffer. After each wash, wait for 1 minute before inverting the plate on absorbent paper to remove any residual buffer.
Peroxidase-conjugated AffiniPure Goat anti-mouse IgG (H+L) antibody at 1/5000 was diluted into the blocking buffer and 100 μL were added in each well. The plate was sealed and incubated for 1 hour at RT.

• Revelation:

Wells were washed 4 times with 200 μL/well of the washing buffer. After each wash, wait for 1 minute before inverting the plate on absorbent paper to remove any residual buffer. 100 μL of the TMB solution were added in each well. The plate was sealed and incubated for 30 minutes at RT in the dark.

• Stop solution:

100 μL of the stop solution were added in each well and the plate was slowly agitated for 5 seconds to homogenize the solution.

• Plate reading:

The optical density is measured using the GloMax Explorer® OD reader at 450nm.

$$\text{Corrected OD450nm} = \text{OD450nm of measured well} - \text{OD450nm of 0 μg/mL Ab well (blank)}$$

The curve obtained with the GN_mAb_Env01 dilution range consisted in a classical sigmoid (Figure 6) presenting a nice linearity phase between induction and saturation plateau. The EC50 calculated here is equal to 1.44 ng/mL.

EXAMPLE 5.3: Immunofluorescence (IF) cytological profiles obtained with GN mAb Env01 antibody.

HEK-293 transfected cells expressing HERV-W ENV and non-transfected cells were cultured on 8 wells Lab-Tek culture slides (Nunc, C7182). 48h after transfection, cells are washed once with 200 μL of Phosphate-Buffered Saline 1X (prepared from PBS 10X pH 7,4 Gibco, 70011-036) and fixed in 4% paraformaldehyde solution (Alfa Aesar, J61899) for 15 minutes. Cells were then washed three times with 200 μL of PBS 1X and permeabilized with 200 μL of PBS 1X supplemented with 0,2 % Tween 20 (Sigma, P7949) for 15 minutes. After 30 minutes of incubation in 200 μL of blocking

solution made with PBS 1X, 0,2% Tween 20 and 2,5 % horse serum (ATCC, 30-2040), cells were incubated with 3, 10 or 30 $\mu$g/mL of GN_mAb_Env01 (primary antibody) diluted in blocking solution during 1 hour. After 3 washes with 200 $\mu$L of PBS 1X, cells were incubated for 1 hour with 4 $\mu$g/mL Alexa Fluor 488 goat anti-mouse IgG antibody (ThermoFisher, A11029) diluted in blocking solution. After 3 washes with 200 $\mu$L of PBS 1X, cells were counterstained with DAPI/anti-fade mounting medium (Vectashield, H-1500). Microscopy was performed with a Zeiss AXIO Scope A.1 microscope, equipped with Zeiss AxioCam MRm camera. Composite images are created using imaged software.

As shown in **Figure 7 A,** the GN_mAb_Env01 antibody provided excellent fluorescent immunolabelling of cells expressing HERV-W ENV from 3 $\mu$g/mL. The specificity of the antibody was ascertained by the absence of a fluorescent signal in cultures of non-transfected cells (whatever the concentration of GN_mAb_Env01 tested). The absence of signal observed in the well of HERV-W ENV transfected cells that did not receive GN_mAb_Env01 validates that the signal observed did not come from a non-specific interaction of the fluorescent secondary antibody on the cells. **Figure 7 B** showed all the cells present in the well by fluorescent labelling of the nuclei with DAPI. This labelling confirms the presence in comparable quantity and distribution in all the wells presented here.

EXAMPLE 5.4: Immunohistochemical (IHC) profiles obtained with GN mAb Env01 antibody.

Paraffin embedded COVID-19 brain tissue section slides from necropsies of COVID-19 patients, previously fixed in 4% paraformaldehyde, were rehydrated by successive bath of toluene, degreasing concentration of ethanol and 1X PBS. Endogenous peroxydases were inhibited in a 30 min bath of 4% H2O2. Permeabilization was performed during 5-10 min in 1X PBS+0.2% Tween 20 and the non-specific sites interaction were blocked by an incubation in 3% horse serum in 1X PBS+0.2% Tween 20 during 30 min at RT. Primary antibodies (anti-HERV-W ENV GN_mAb_Env01 and anti-Iba-1, a specific marker of microglia) were incubated overnight at 4°C and secondary antibodies were incubated during 45 min at RT. Revelation was performed using AEC kit (Vector, SK-4200) following supplier indications. Nuclei were counter-stained during 3 min with Harris hematoxylin (filtrated and 3-fold diluted) before a quick rinse in water. Slides were mounted using Fluoromount (Southern Biotech).

As shown in **Figure 8,** the GN_mAb_Env01 antibody allowed an excellent labelling of cells expressing HERV-W ENV on paraffin-embedded human tissue slides. Here, it has been possible to see that cells expressing HERV-W ENV (**Figure 8A**) also expressed the microglia-specific marker Iba-1 (**Figure 8 B**), using adjacent paraffin-embedded tissue slides from the brains of patients who had died of COVID-19. Study of the morphology of HERV-W ENV and Iba-1 positive cells confirmed the co-expression of these two proteins.

## EXAMPLE 6: Production of anti-HERV-K ENV antibodies

Iimmunization of mice, lymphocyte hybridization, hybridoma cloning, antibody production and purification were followed by ELISA screening at every step.

EXAMPLE 6.1: Immunization protocol and generation of hybridomas

3 female mice OF1 (S1, S2 and S3) were immunized with his-SUMO tagged HERV-K ENV-SU protein produced in E.coli (Mybiosource, USA, ref. MBS1391552) according to the MO.CEL.041 protocol of R.A.D.® immunization (Rapid Antibody Development; Biotem, Apprieu, France). 10 days after immunization, all sera were tested by ELISA with coating of HERV-K ENV-SU protein and the 3 mice showed a strong immune response against this antigen. 13 days after immunization, mice were sacrificed and immune cells from lymph nodes were collected. A single lymphocyte fusion with S1+S2+S3 mice was performed according to MO.CEL.042 protocol of R.A.D.®(Biotem, Apprieu, France) in presence of polyethylenglycol (PEG) as fusing agent with the following parameters:

| | |
|---|---|
| - Number of myeloma cells: | $107 \times 10^6$ ¢ |
| - Number of lymphocytic cells: | $420 \times 10^6$ ¢ |
| - Ratio: | 1/3.9 |
| - Number of 96-well plates: | 5 plates at 100,000 cells/well (n°1 to 5) |
| | 15 plates at 50,000 cells/well (n°6 to 20) |
| - Number of wells analyzed: | 400 (~21%) |

Cells were cultivated at 37°C, 5% $CO_2$ for 21 days in the following culture medium: Dulbecco's Modified Eagle's (DMEM, SIGMA, D5671), 1X Hypoxanthine Aminopterin Thymidine (HAT, SIGMA, H0262-10VL), 20% fetal calf serum (FCS, PAA, A15-251), 4 mM L-Glutamine (SIGMA, G7513), 1% penicillin/streptomycine (SIGMA, P0781).

After fusion, hybridoma supernatants were screened by ELISA with coating of 1 $\mu$g/mL HERV-K ENV-SU protein according to protocol MO.ELI.014 (Biotem, Apprieu, France). A second round of ELISA (confirmation ELISA) was performed with coating of 1 $\mu$g/mL HERV-K ENV-SU, E.Coli lysate (XL1-Blue MRF, Stratagene ; negative control) or without antigen (analysis of "sticky" antibodies) and 58 hybridoma were pre-selected. Out of these 58, 5 hybridoma were selected for cloning and isotyping respectively performed according to MO.CEL.010 and MO.ELI.012 protocols (Biotem, Apprieu, France).

Clones supernatants were screened by ELISA with coating of 1 $\mu$g/mL HERV-K ENV-SU and confirmation ELISA was performed with coating of 1 $\mu$g/mL HERV-K ENV-SU, E.Coli lysate (XL1-Blue MRF, Stratagene ; negative control) or without antigen (analysis of "sticky" antibodies). After 1 or 2 rounds of cloning, 2 clones and 8 subclones were selected and cryopreserved. A second round of screening was performed on previous lymphocyte hybridization with the following parameters:

|  |  |
|---|---|
| - Number of 96-well plates: | 11 plates at 200,000 cells/well (n°21 to 31) |
|  | 6 plates at 150,000 cells/well (n°32 to 37) |
|  | 3 plates at 100,000 cells/well (n°38 to 40) |
| - Number of wells analyzed: | 484 (~25%) |

484 hybridoma supernatants were screened by indirect ELISA according to MO.ELI.014 protocol (Biotem, Apprieu, France) with coating of 1 $\mu$g/mL HERV-K ENV-SU protein or HEK 293T cells transfected with plasmid coding for HERV-K ENV protein and sonicated using a ultrasonic device. After a second round of confirmation ELISA, 137 hybridoma appeared to be positive for detecting HERV-K ENV-SU protein and 9 of these 137 were also positive on sonicated cells. One was particularly selected to generate GN_mAb_Env-K01antibody, as presented in the present test for HERV-K ENV quantification.

EXAMPLE 6.2: Production and purification of anti-HERV-K ENV antibodies

GN_mAb_Env-K01 was produced and purified from its selected hybridoma.

The hybridoma culture was amplified in the following culture medium: DMEM (SIGMA, D5671), 15% of low IgG FCS (Life Technologies, 16250), 4mM L-Glutamine (SIGMA, G7513), 1% HES (SIGMA H6020), 1% penicillin/streptomycine (SIGMA, P0781). For each culture, 500 mL to 1 L of supernatant were produced in Hyperflask (Corning, 10030) at 37°C, 5% CO2 within 10+/-1 days according to MO.CEL.049 protocol (Biotem, Apprieu France). Antibodies were purified by protein A chromatography with MabSelect Xtra antibody purification chromatography resin (protocol MO.PUR.018 ; Biotem, Apprieu France).

Antibody concentrations were measured by optical density at 280 nm using BioSpectrometer from Eppendorf. Purity of antibodies (> 95%) was determined by densitometry analysis on SDS PAGE electrophoresis (MO.PUR.015 protocol, Biotem, Apprieu, France). Bioburden was analyzed on blood agar after filtration of antibodies on a 0.22 $\mu$m filter. Antibodies were stored in the following buffer: PBS pH 7,4 (NaCl 137 mM, KCl 2,7 mM, $Na_2HPO_4$ 10 mM, $KH_2PO_4$ 2 mM).

The antibody GN_mAb_Env-K01 comprises each of the complementary-determining regions (CDRs) set forth in SEQ ID NO: 9, SEQ ID NO: 10, SEQ ID NO: 11 SEQ ID NO: 12, SEQ ID NO: 13 and SEQ ID NO: 14.

More particularly, said antibody comprises:

- a light chain comprising a light chain variable region (VL) which comprises three complementarity determining regions: CDR-L1 as set forth in SEQ ID NO:9, CDR-L2 as set forth in SEQ ID NO:10 and CDR-L3 as set forth in SEQ ID NO:11, and
- a heavy chain comprising a heavy chain variable region (VH) which comprises three complementarity determining regions: CDR-H1 as set forth in SEQ ID NO:12, CDR-H2 as set forth in SEQ ID NO:13 and CDR-H3 as set forth in SEQ ID NO:14.

**EXAMPLE 7: Characterization of GN_mAb_Env-K01 anti-HERV-K ENV antibody on glycosylated and deglycosylated HERV-K ENV**

GN_mAb_Env-K01 was evaluated on Simple Western assays (see **Figure 9**). This immunoassay was performed in denaturing conditions. Our results showed that GN_mAb_Env-K01 detected both glycosylated or deglycosylated forms of HERV-K ENV.

**EXAMPLE 8: Detection of HERV-W ENV in human biological fluids**

EXAMPLE 8.1: HERV-W ENV detection in human serum with a **"detection-only"** 3-steps test for HERV-W ENV

This sub-section describes the old test, which could only be used to determine whether a sample was positive or negative for the presence of HERV-W ENV ("detection-only test") and which could not be used to make a universal quantification diagnostic kit with a built-in calibration curve.

HERV-W ENV, as described by its sequence and highly homologous others, is not physiologically expressed in human tissues, except for Syncytin-1 encoded by a modified HERV-W envelope gene with characteristic sequence differences and functional domains as well described, along with sufficiently divergent epitopes for our specific anti-HERV-W antibodies that did not cross react with Syncytin-1 (Charvet et al., 2021).

As observed so far, HERV-W ENV is detected in patients with multiple sclerosis, type 1 diabetes, schizophrenia, CIDP, acute or long COVID-19 and fibromyalgia.

**Materials and Methods: Detection-only test with "3 steps test"**

**Table 5: Biological and chemical reagents and materials**

| Material or Reagent name | Reagent type | Reference | Supplier |
|---|---|---|---|
| recombinant HERV-W ENV | Recombinant protein produced in E. coli | n/a | GTP Bioways |
| GN_mAb_Env01 | Murine monoclonal antibody | n/a | GNI |
| RIPA 1X | Cell lysis buffer | R0278 | Merck |
| Fos Choline 16 | Detergent well suited for the solubilization, stabilisation, and purification of membrane proteins | F316S-1GM | Anatrace |
| cOmplete™ ULTRA 25X | Protease inhibitor cocktail | 5892791001 | Roche |
| Protein Deglycosylation Mix II | Protein Deglycosylation enzyme and buffer | P6044S | New England Biolabs |
| Amicon Ultra-0.5 Centrifugal Filter Unit | Protein purification (100 kDa cutoff) | UFC510096 | Merck-Millipore |

**Table 6: Simple Western materials and reagents**

| Material or Reagent name | Reference | Supplier |
|---|---|---|
| Simple Western Automated Western Blot instrument | n/a | ProteinSimple |
| 66-440 kDa Separation Module | SM-WO08 | ProteinSimple |
| Secondary Streptavidin HRP | 043-459-2 | ProteinSimple |
| Anti-Mouse Secondary HRP Antibody | 042-205 | ProteinSimple |
| Antibody Diluent 2 | 042-203 | ProteinSimple |
| Peroxide | 043-379 | ProteinSimple |
| Luminol-S | 043-311 | ProteinSimple |
| Sample Buffer 10X | 042-195 | ProteinSimple |

**Sample preparation step 1:** Protein extraction

50 $\mu$L of human serum is incubated for 2 h at 25°C with gentle agitation in presence of Fos Choline 16 (Anatrace, F316S-1GM) at 1% final concentration. Fos Choline 16 was previously resuspended at 10% in 1X RIPA buffer (R0278, Merck) complemented with protease inhibitor cocktail (5892791001, Roche). Supernatants are collected after 10 min of centrifugation at 10,000 $\times$ g.

**Sample preparation step 2:** Deglycosylation

70 μg of total protein extract diluted in 40 μL of nuclease-free water are deglycosylated using the Protein Deglycosylation Kit from New England Biolabs (P6044S). 5 μL of denaturing buffer (B6045SVIAL) are added and the mix is incubated 10 min at 75 °C. Then, after cooling on ice, 5 μL of enzyme mix (P6044SVIAL) are added and incubated for 16 h at 37 °C.

**Sample preparation step 3:** Protein Size Separation Using Amicon filter unit

10 μL of deglycosylated proteins are diluted in 500 μL of 1X RIPA buffer and loaded in Amicon Ultra-0.5 Centrifugal Filter Unit (UFC510096, Merck-Millipore). High molecular weight proteins are enriched on column filter by 30 min centrifugation at 14,000 ×g and concentrates are collected.

**Technological analysis platform:** Simple Western Automated Western Blot

The amount of HERV-W ENV antigen is assessed on Simple Western (Jess, Leo, Wes or Abby) devices using Simple Western technology, an automated capillary-based size sorting and immunolabeling system (ProteinSimple™, Bio-Techne). All procedures are performed with manufacturer's reagents according to their manual. Pure protein concentrate (concentrate from Amicon filter unit) is mixed with fluorescent master mix and heated at 95 °C for 5 min. The samples, blocking reagent, wash buffer, primary antibody (20 μg/mL murine GN_mAb_Env01 antibody) and their respective ready to use secondary reagents (ready to use Anti-Mouse Secondary HRP Antibody), and chemiluminescent substrate were dispensed into the microplate. Samples are loaded in duplicates or triplicates into individual capillaries on a 25 capillary cartridge (66-440 kDa Simple Western separation module, SM-WO08, Bio-Techne). Protein separation and immunodetection is performed automatically on individual capillaries using default settings. The global signal (area under the curve, AUC) containing the target hexamer HERV-W ENV (electrophoregram peak about 440 kDa) is measured using Compass software for Simple Western (Jess, Leo, Wes or Abby) devices settings and raw data recording (ProteinSimple/Bio-Techne).

**HERV-W ENV detection-only test:** inter-plates normalization and S/N calculation

The purified HERV-W ENV recombinant protein produced in *E. coli* is deposited in triplicates on each Simple Western plate at a concentration of 250 ng/mL (dilution in 0.1X Sample Buffer). The detection is performed using 20 μg/mL GN_mAb_Env01 as primary antibody and anti-mouse secondary HRP antibody. The mean of the raw AUC obtained for the peak corresponding to recombinant HERV-W ENV monomer (apparent molecular weight 45-50 kDa on 66-440 kDa separation matrix) is used to calculate the normalization factor to be applied to each plate using the formula: *mean of the AUC of the triplicates of "recombinant protein" on the reference plate / mean of the AUC of the triplicates of "recombinant protein" on the test plate*. The normalization factor thus calculated is applied to all the raw values (400-440 kDa peak AUC) of the plate studied to obtain normalized AUC. Signal/Noise (S/N) ratio is obtained by dividing normalized AUC of the serum to be tested by the normalized mean AUC + 2 SD calculated with sera from a panel of healthy blood donors. HERV-W ENV positivity is then determined by S/N superior to 1.

This detection-only test:

- lasts two days because of the time of the preparation of the sample in 3 steps ;
- requires a panel of serum samples from healthy individual in order to determine the non-specific background signal (noise/N) generated by negative sera at the same MW level as the specific peak of the HERV-W ENV oligomer. This matrix effect is variable with sera and requires a statistical calculation of the threshold for the non-specific signal generated by normal sera from various origins (N= Mean + 2 SD from AUC of sera analyzed within a panel of healthy donors) as control;
- Has a significant background noise which obliges the calculation of S/N ratio;
- The result is only a yes or no conclusion on the presence of HERV-W ENV in the tested sample, it does not allow to quantify HERV-W ENV in the sample.
- Nor does it allow to detect low-positive samples when taken in the range of the possible noise (≤N), therefore qualified as negative when they are not (false negative results of this test), therefore precluing precision medicine since missing these cases.
- It does not allow to validate significant variations between samples of the same patient at different timepoints, for a follow-up, therefore precluding individualized medicine.

EXAMPLE 8.2: HERV-W ENV detection in human serum with a novel test providing an absolute quantification and a one-step test for HERV-W ENV

**Table 7: Biological and chemical reagents and materials**

| Material or Reagent name | Reagent type | Reference | Supplier |
|---|---|---|---|
| recombinant HERV-W ENV | Recombinant protein produced in E. coli | n/a | GTP Bioways |
| bTmAb | Biotin-conjugated Temelimab humanized monoclonal antibody | n/a | Biotem |
| cOmplete™ ULTRA 25X | Protease inhibitor cocktail | 5892791001 | Roche |
| Mem-PER™ Plus Membrane Protein Extraction Kit | Protein Purification and Isolation reagents | 89842 | ThermoFisher Scientific |

**Table 8: Simple Western materials and reagents**

| Material or Reagent name | Reference | Supplier |
|---|---|---|
| Simple Western Automated Western Blot instrument | n/a | ProteinSimple |
| 66-440 kDa Separation Module | SM-WO08 | ProteinSimple |
| Secondary Streptavidin HRP | 043-459-2 | ProteinSimple |
| Antibody Diluent 2 | 042-203 | ProteinSimple |
| Peroxide | 043-379 | ProteinSimple |
| Luminol-S | 043-311 | ProteinSimple |
| Sample Buffer 10X | 042-195 | ProteinSimple |

**Sample preparation:**

25 μL of each serum are incubated for 10 min with 250 μL of Permeabilization buffer (part of the Mem-PER Plus Membrane Protein Extraction Kit, 89842, ThermoScientific) supplemented with protease inhibitor cocktail (final concentration: 1X ; 5892791001, Roche). After 15 min of centrifugation at 14 000 g at room temperature, supernatants are eliminated, pellets are resuspended with 100 μL of Solubilization buffer (part of the Mem-PER Plus Membrane Protein Extraction Kit, 89842, ThermoScientific) supplemented with protease inhibitor cocktail (final concentration: 1X ; 5892791001, Roche) and incubated 30 min at room temperature. After 15 min of centrifugation at 14 000 g at room temperature, supernatants are collected.

**Simple Western Automated Western Blot**

The amount of HERV-W ENV antigen is assessed on Simple Western (Jess, Leo, Wes or Abby) devices using Simple Western technology, an automated capillary-based size sorting and immunolabeling system (ProteinSimple™, Bio-Techne). All procedures are performed with manufacturer's reagents according to their manual. Pure protein extract (final supernatant of Mem-PER extraction) is mixed with fluorescent master mix and heated at 95 °C for 5 min. The samples, blocking reagent, wash buffer, primary antibody (100 μg/mL Biotinylated Temelimab antibody) and their respective ready to use secondary reagents (ready to use Streptavidin HRP), and chemiluminescent substrate were dispensed into the microplate. Samples are loaded in duplicates or triplicates into individual capillaries on a 25 capillary cartridge (66-440 kDa Simple Western separation module, SM-WO08, Bio-Techne). Protein separation and immunodetection is performed automatically on individual capillaries using default settings. The global signal (area under the curve, AUC) containing the target hexamer HERV-W ENV (electrophoregram peak about 440 kDa) is measured using Compass software for Simple Western (Jess, Leo, Wes or Abby) devices settings and raw data recording (ProteinSimple/Bio-Techne).

Novel conditions and tests providing a complete and reproducible extraction combined by further novel conditions for an absolute quantification of HERV-W ENV hexamer in human serum:

- Thanks to the findings allowing hexamerization of the recombinant protein as detailed in EXAMPLE 3.2, a 6-points hexameric range of recombinant HERV-W ENV (0-12.5-25-50-100-200 ng/mL) is prepared by 2-fold serial dilution in Solubilization Buffer (part of the Mem-PER Plus Membrane Protein Extraction Kit, 89842, ThermoScientific) and kept at room temperature few minutes before being processed for Simple Western analysis. This range is deposited on each Simple Western plate used to analyze a human serum. 440 kDa peakAUC corresponding to recombinant HERV-W ENV hexamer is measured for each range point. Non-linear logarithmic analysis is employed to obtain standard curve. Interpolation of this curve enables to convert HERV-W ENV hexamerAUC of serum sample into µg/mL of HERV-W ENV.

- In addition, the novel test also uses Biotinylated temelimab, an anti-HERV-W ENV specific humanized IgG4 recombinant antibody, with Streptavidin-HRP instead of GN_mAb_Env01 with anti-mouse-HRP in the previous test. This very significantly reduced the background noise from the final detection step for the quantification of the specific signal. This also resulted in a strong improvement of HERV-W ENV hexamer definition on the electrophoregram thereby made much easier to analyze without major baseline adjustments.

EXAMPLE 8.3: Comparison between detection-only "3-steps" and absolute quantitative "1-step" tests for HERV-W ENV detection in human serum

**Table 9: Brief comparison between immunoassay technologies**

|  | ELISA | WESTERN BLOT | DETECTION ONLY SIMPLE WESTERN PROTOCOL | QUANTITATIVE SIMPLE WESTERN PROTOCOL |
|---|---|---|---|---|
| Time consuming (sample + preparation + analyze) | few hours | 2 days | 3 days | few hours |
| matrix effect / antigen masking | yes | no (but background) | no (but background) | no |
| Steps introducing variability (e.g., enzyme digestion kinetics, concentration on AMICON column) | no | yes | yes | no |
| Oligomerization of recombinant protein for concentration range | no | no | no | yes |
| Quantification | Non reproducible detection | Detection-only | detection-only (S/N >1 positivity) | Absolute (standard curve: hexamer) |
| Developability | no | no | no | yes |

The novel quantitative test for one-step serum sample preparation allowed complete and reproducible extraction of HERV antigen Followed by Simple Western quantification.

The comparison between the previous test using the simple western technology and the combination of novel protocols revealed critical improvements now allowing HERV-ENV quantification in a routine test format for in vitro-diagnostics:

- Sample preparation lasted two days in the previous test, whereas in the new test takes only 2.5 hours (See Figure 10)
- the previous test had the sample preparation protocol requiring 3 steps (extraction, deglycosylation and concentration on a column to be centrifuged), whereas the new version of the protocol only involves protein extraction in a buffer.
- The previous test included 2 non-calibratable steps (inducing uncontrollable variations): deglycosylation (an enzymatic reaction which cannot be calibrated for reproducible quantitative results) and the concentration column on which not all proteins with molecular weights below the cut-off are eliminated and which required to spin down the deglycosylation medium with variable efficiency in the final separation and collection of the target antigen.
- The single extraction step of the new sample preparation protocol for the new test is a protein extraction with the Mem-PER kit, which is easy to calibrate.

EXAMPLE 8.4: Using the humanized recombinant antibody temelimab for a highly sensitive HERV-W ENV hexamer detection without significant non-specific noise in the novel Simple Western protocol: a diagnostic test with complete antigen extraction and absolute quantification.

Using the directly biotinylated humanized recombinant antibody temelimab allows to eliminate the background noise and allows a much greater sensitivity than the procedure using the murine antibody and a secondary antibody from the previous test.

• HERV-W ENV hexamer definition in novel Simple Western protocol

The novel test also uses biotinylated temelimab with Streptavidin-HRP instead of GN_mAb_Env01 with anti-mouse-HRP in the previous test. This very significantly reduced the background noise from the final detection step for the quantification of the specific signal. This also resulted in a strong improvement of HERV-W ENV hexamer definition on the electrophoregram thereby made much easier to analyze without major baseline adjustments. This avoided misinterpretation or variability of interpretation between experimenters, which is key to the development of a diagnostic test to be used by various technicians in many different places.

Comparison between previous and novel tests was performed on 12 human sera.

Both sera preparations analyzed on Jess device show a good but not perfect correlation with r > 0.78 (Figure 12). The same analysis was performed on a second site with Simple Western instrument and again both sera preparations show a relative correlation with r > 0.66 with a moderate p value equal to 0.0190 (*).

• Assay sensitivity of the novel Simple Western test

The use of this novel test also revealed that the Streptavidin-HRP detection module was more sensitive than the previously used Anti-Human HRP detection module (STRP-HRP), but also required the use of biotinylated temelimab (bTmAb) instead of a murine primary antibody (Figure 13).

• Absolute quantification (novel test) rather than detection-only (previous test) with simple Western platform.

It was observed that the "Solubilization buffer" reagent contained in the "Mem-PER" protein extraction kit was capable of strongly inducing auto-oligomerization of HERVs-ENV and HERVs-ALT proteins, provided they had a sufficiently hydrophobic end. The new version of the test enables us to divert from the initial use of the "Solubilization buffer" to use it as an "oligomerization buffer" in order to prepare standard ranges of oligomerized recombinant protein corresponding to the antigenic form observed in patients' biological fluids. This allows absolute quantification with the new test (Figures 14, 15 and 16).

EXAMPLE 8.5: Newly discovered protocol for the complete and reproducible oligomerization of the purified HERV-W ENV recombinant monomeric E. coli protein.

The following study on CSFs from multiple sclerosis (MS) patients evidences the significant contribution of recombinant hexamers used for a standard curve to the quantification of HERV-W ENV protein.

**Materials and Methods**

**Table 10: Device, reagents, antibodies and recombinant antigens**

| Device | Reference | Supplier | |
|---|---|---|---|
| Jess | Jess | ProteinSimple | |
| 25 capillary cartridge 66-440 kDa matrix | SM-WO08 | ProteinSimple | |
| **Reagents** | **Reference** | | **Supplier** |
| Solubilization buffer (from Mem-PER extraction kit) | Mem-PER Plus Membrane Protein Extraction Kit (ref: 89842) | | ThermoScientific |
| Protease inhibitor cocktail | 5892791001 | | Roche |

(continued)

| Antibody | Reference | Supplier | Isotype | Host |
|---|---|---|---|---|
| Anti-HERV-W ENV detection antibody | Temelimab (batch: T1940422-A) | GeNeuro | IgG4 | humanized |
| anti-human HRP-coupled secondary antibody | DM-005 | ProteinSimple | Not specified | |

Sample preparation and Simple Western analysis:

50 µL of each CSF were incubated for 10 min with 50 µL of Permeabilization buffer (part of the Mem-PER Plus Membrane Protein Extraction Kit, 89842, ThermoScientific) supplemented with protease inhibitor cocktail (5892791001, Roche).

The amount of HERV-W ENV antigen was assessed on Simple Western technology device (Wes, Jess, Leo, Abby), an automated capillary-based size sorting and immunolabeling system (ProteinSimpleTM). Briefly, prepared CSF were mixed with fluorescent master mix (ProteinSimple, USA) and heated at 95 °C for 5 min. The samples, blocking reagent, wash buffer, 20 µg/mL of primary antibody: humanized temelimab (T1940422-A, GeNeuro) and its ready to use detection module (DM-005, ProteinSimple: anti-human HRP-coupled secondary antibody), and chemiluminescent substrate were dispensed into the microplate. Samples were loaded in triplicates into individual capillaries on a 25 capillary cartridge (66-440 kDa Jess separation module, SM-WO08, ProteinSimple). Protein separation and immunodetection was performed automatically on individual capillaries using default settings. The global signal (AUC) containing the target hexamer HERV-W ENV (electrophoregram peak about 400 kDa) was measured using Compass software for Simple Western devices settings and raw data recording (ProteinSimple/Biotechne, USA).

Raw data generation and analysis:

HERV-W ENV antigen in biological fluids is organized in hexameric form providing solubility properties to this insoluble monomeric protein (more details in Charvet et al., 2021). The apparent molecular weight of the hexameric HERV-W ENV is 350-440 kDa and the area under curve (AUC) of the peak corresponding to this peak is proportionate to the quantity of targeted protein present in the extract from the sample. In the case of CSF, it has been found that the diverted use of the Solubilization buffer from the MEM-PER kit (Thermofischer, USA) alone was also appropriate for the sample preparation before immunoanalysis by SimpleWestern. This is further combined with calculation of an accurate antigen concentration when using the newly obtained hexameric recombinant for the standard curve. **Figure 17** presents representative example of negative and positive sample and how the AUC value is determined.

The absolute quantification dosage was performed as previously described in "EXAMPLE 8.1". Indeed, dilution range of hexamerized recombinant protein (dilution in solubilization buffer form Mem-PER kit) provided a nice linear curve allowing to quantify HERV-W ENV in human CSF (see method previously described in **Figure 16**). For example, in **Figure 17,** the HERV-W ENV positive CSF from **Figure 17 A** contained 1250 pg/mL CSF when a residual signal corresponding to background noise was barely seen in the negative one presented in **Figure 17 B.**

**REFERENCES**

- Belshaw R, Pereira V, KatzourakisA, Talbot G, Paces J, Burt A, Tristem M. Long-term reinfection of the human genome by endogenous retroviruses. Proc Natl Acad Sci USA. 2004 Apr 6;101(14):4894-9. doi: 10.1073/pnas.0307800101. Epub 2004 Mar 25. PMID: 15044706; PMCID: PMC387345.Dewannieux et al., 2006 Genome Research.

- Gröger V, Wieland L, Naumann M, Meinecke AC, Meinhardt B, Rossner S, Ihling C, Emmer A, Staege MS, Cynis H. Formation of HERV-K and HERV-Fc1 Envelope Family Members is Suppressed on Transcriptional and Translational Level. Int J Mol Sci. 2020 Oct 23;21(21):7855. doi: 10.3390/ijms21217855. PMID: 33113941; PMCID: PMC7660216.

- Beimforde N, Hanke K, Ammar I, Kurth R, Bannert N. Molecular cloning and functional characterization of the human endogenous retrovirus K113. Virology. 2008 Feb 5;371(1):216-25. doi: 10.1016/j.virol.2007.09.036. PMID: 18076964. Hanke et al. 2009.

- Fatima, S., Sen, P., Sneha, P. et al. Hydrophobic Interaction Between Domain I of Albumin and B Chain of Detemir May Support Myristate-Dependent Detemir-Albumin Binding. Appl Biochem Biotechnol 182, 82-96 (2017).

- Charvet B, Pierquin J, Brunel J, Gorter R, Quétard C, Horvat B, Amor S, Portoukalian J, Perron H. Human Endogenous Retrovirus Type W Envelope from Multiple Sclerosis Demyelinating Lesions Shows Unique Solubility and Antigenic Characteristics. Virol Sin. 2021 Oct;36(5):1006-1026. doi: 10.1007/s12250-021-00372-0. Epub 2021 Mar 26. PMID: 33770381; PMCID: PMC8558138.

- Komurian-Pradel F, Paranhos-Baccala G, Bedin F, Ounanian-Paraz A, Sodoyer M, Ott C, Rajoharison A, Garcia E, Mallet F, Mandrand B, Perron H. Molecular cloning and characterization of MSRV-related sequences associated with retrovirus-like particles. Virology. 1999 Jul 20;260(1):1-9. doi: 10.1006/viro.1999.9792. PMID: 10405350.

- Perron H, Jouvin-Marche E, Michel M, Ounanian-Paraz A, Camelo S, Dumon A, Jolivet-Reynaud C, Marcel F, Souillet Y, Borel E, Gebuhrer L, Santoro L, Marcel S, Seigneurin JM, Marche PN, Lafon M. Multiple sclerosis retrovirus particles and recombinant envelope trigger an abnormal immune response in vitro, by inducing polyclonal Vbeta16 T-lymphocyte activation. Virology. 2001 Sep 1;287(2):321-32. doi: 10.1006/viro.2001.1045. PMID: 11531410.

**Claims**

1. Quantitative detection method of HERV ENV in a sample to be analyzed, with a capillary immunoelectrophoresis device and based on an anti-HERV ENV biotinylated antibody and a marker conjugated to streptavidin, as compared to a standard of recombinant HERV ENV protein.

2. Quantitative detection method of HERV ENV according to claim 1, wherein HERV ENV is HERV-W ENV and said anti-HERV ENV biotinylated antibody is an anti-HERV-W ENV biotinylated antibody.

3. Quantitative detection method of HERV ENV according to claim 2, wherein said anti-HERV-W ENV antibody comprises each of the complementary-determining regions (CDRs) set forth in SEQ ID NO: 1, SEQ ID NO: 2, SEQ ID NO: 3 SEQ ID NO: 4, SEQ ID NO: 5 and SEQ ID NO: 6.

4. Quantitative detection method of HERV ENV according to claim 2 or 3, wherein said anti-HERV-W ENV antibody is a humanized antibody.

5. Quantitative detection method of HERV ENV according to any of claims 2 to 4, wherein said anti-HERV-W ENV antibody comprises:

   - a light chain comprising a light chain variable region (VL) as depicted in SEQ ID NO:7, and
   - a heavy chain comprising a heavy chain variable region (VH) as depicted in SEQ ID NO:8.

6. Quantitative detection method of HERV ENV according to claim 1, wherein HERV ENV is HERV-K ENV and said anti-HERV ENV biotinylated antibody is an anti-HERV-K ENV biotinylated antibody.

7. Quantitative detection method of HERV ENV according to claim 6, wherein said anti-HERV-K ENV antibody comprises each of the complementary-determining regions (CDRs) set forth in in SEQ ID NO: 9, SEQ ID NO: 10, SEQ ID NO: 11 SEQ ID NO: 12, SEQ ID NO: 13 and SEQ ID NO: 14.

8. Quantitative detection method of HERV ENV according to any of claims 1 to 7, wherein the sample is serum or plasma.

9. Quantitative detection method of HERV ENV according to any of claims 1 to 8, wherein marker is a peroxidase with a light-generating substrate.

10. Quantitative detection method of HERV ENV according to any of claims 1 to 9, wherein the standard of recombinant HERV ENV protein is a calibration curve obtained from a range of known concentrations of recombinant HERV ENV proteins.

11. Quantitative detection method of HERV ENV according to claim 10, wherein the recombinant HERV ENV proteins are under oligomeric form, in particular obtained after incubation of monomeric HERV ENV proteins in a solubilization buffer comprising :

- Bicine (pH7.5 buffer)
- A zwitterionic detergent, such as CHAPS, 3-[(3-cholamidopropyl)diméthylammonio]-1-propanesulfonate, C32H58N2O7S) and/or a detergent with a polar extremity and a long aliphatic chain, such as LPG [Lyso PG 1-myristoyl-2-hydroxy-sn-glycero-3-phospho-(1'-rac-glycerol)] or fos-Choline 16 ( n-Hexadecylphosphocholine, C21H46NO4P)
- Optionally a dilution or a protein extract from serum, such as human serum or fetal calf serum,
- a protease inhibitor or a mix of protease inhibitors.

12. Kit for a quantitative detection method of HERV ENV comprising:

- An anti-HERV ENV biotinylated antibody, and
- A marker conjugated to streptavidin.

13. A humanized anti-HERV-W ENV biotinylated antibody comprising :

- a light chain comprising a light chain variable region (VL) as depicted in SEQ ID NO:7, and
- a heavy chain comprising a heavy chain variable region (VH) as depicted in SEQ ID NO:8.

# Figure 1

Signal peptide

Transmembrane domain

N-term Ectodomain Cytoplasmic tail C-term

# Figure 2

Figure 3

EP 4 764 497 A1

38

## Figure 4

# Figure 5

**Figure 6**

**Figure 7**

# Figure 8

# Figure 9

1: HEK-N.T.
2: HEK-K113
3: d-HEK-N.T.
4: d-HEK-K113
\*: Deglycosylated ENV K113
\*\*: Glycosylated ENV K113

# Figure 10

| Sample preparation Processing time | | 3-step Protocol | 1-step Protocol |
|---|---|---|---|
| 2 hrs 30 min | Working Day 1 | RIPA + FC-16 Protein Extraction | Mem-PER Protein Extraction |
| 16 hrs 30 min | Night | Deglycosylation | |
| 45 min | Working Day 2 | High MW Proteins Enrichment | |

**Figure 11**

## Figure 12

**A**

Site A / Jess instrument

| Pearson r | |
|---|---|
| r | 0.7887 |
| 95% confidence interval | 0.3923 to 0.9380 |
| R squared | 0.6220 |
| | |
| P value | |
| P (two-tailed) | 0.0023 |
| P value summary | ** |
| Significant? (alpha = 0.05) | Yes |
| | |
| Number of XY Pairs | 12 |

**B**

Site B / Wes intrument

| Pearson r | |
|---|---|
| r | 0.6622 |
| 95% confidence interval | 0.1425 to 0.8957 |
| R squared | 0.4385 |
| | |
| P value | |
| P (two-tailed) | 0.0190 |
| P value summary | * |
| Significant? (alpha = 0.05) | Yes |
| | |
| Number of XY Pairs | 12 |

## Figure 13

Figure 14

## Figure 15

**A**

Simple Linear Regression

r²=0.9973

**B**

Non-linear Logarithmic Fit

r²(weighted)=0.9895

**C**

| rHERV-W ENV | % Recovery of standard curve to analytical fit | |
|---|---|---|
| | Linear regression | Non-linear log fit |
| 800ng/mL | | |
| 400ng/mL | | 87.0% |
| 200ng/mL | 98.5% | 98.6% |
| 100ng/mL | 106.0% | 109.5% |
| 50ng/mL | 104.0% | 111.9% |
| 25ng/mL | 90.9% | 104.4% |
| 12.5ng/mL | 78.5% | 100.1% |
| 6.25ng/mL | | 90.5% |
| 3.125ng/mL | | 97.9% |

**D**

| rHERV-W ENV hexamer | ng/mL |
|---|---|
| LOD | 2.20 |
| LOQ | 8.05 |

## Figure 16

# Figure 17

**Figure 18**

**A**

Protein extract

Antibody diluent

Primary antibody

Secondary antibody

Luminol/Peroxide

Wash buffer

66-440 kDa

Empty wells

Prefilled matrix
polymerization buffer

**B**

Load Matrix

☐ Stacking Matrix   ☐ Separation Matrix

Load Sample

○ High MW   ○ Low MW

Separate

Immobilize

Immunoprobe

Y Target Protein   Y HRP-labeled
  Primary Ab       Secondary Ab

Quantitate

Chemiluminescence

## EUROPEAN SEARCH REPORT

**Application Number**

EP 24 30 7240

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/201908 A1 (GENEURO SA [CH]) 24 October 2019 (2019-10-24) | 1-4,8-12 | INV. G01N33/569 |
| A | * PG12,13,19,25-28; paragraph [0015]; claims 1,2,11,12; sequences 5-10 * | 5-7,13 | ADD. G01N27/447 |
| | ----- | | |
| X | WO 2010/003977 A1 (GENEURO SA [CH]; BERNARD CORINNE [FR] ET AL.) 14 January 2010 (2010-01-14) | 1-5,8-13 | |
| A | * pages 9,10,87; claims 1,6,7; figure 1; sequences 1-6,40,48 * | 6,7 | |
| | ----- | | |
| X | WO 2015/181226 A1 (GENEURO SA [CH]) 3 December 2015 (2015-12-03) | 1-5,8-13 | |
| A | * claims 7,20; sequences 1-8 * | 6,7 | |
| | ----- | | |
| A | US 2010/304359 A1 (EGAN RICHARD LASWELL [US] ET AL) 2 December 2010 (2010-12-02) * paragraphs [0026], [0030], [0207]; claims 4-6 * | 1-13 | |
| | ----- | | **TECHNICAL FIELDS SEARCHED (IPC)** |
| X | EP 3 351 265 A1 (GENEURO SA [CH]) 25 July 2018 (2018-07-25) | 1,6-12 | G01N |
| A | * paragraphs [0003], [0028], [0035], [0081]; claims 2,4; figures 2,5; table 1; sequences 1-6 * | 2-5,13 | |
| | ----- | | |

The present search report has been drawn up for all claims

| Place of search | Date of completion of the search | Examiner |
|---|---|---|
| The Hague | 14 July 2025 | Kosten, Jonas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

EP 24 30 7240

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

Application Number

EP 24 30 7240

The Search Division considers that the present European patent application does not comply with the requirements of unity of invention and relates to several inventions or groups of inventions, namely:

1. claims: 2-5, 13(completely); 1, 8-12(partially)

   The claims in so far as they relate to an anti-HERV-W ENV antibody with CDRs of SEQ-IDs 1-6.
   ---

2. claims: 6, 7(completely); 1, 8-12(partially)

   The claims in so far as they relate to an anti-HERV-K ENV antibody with CDRs of SEQ-IDs 9-14.
   ---

## ANNEX TO THE EUROPEAN SEARCH REPORT
## ON EUROPEAN PATENT APPLICATION NO.

EP 24 30 7240

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2025

| Patent document cited in search report | Publication date | Patent family member(s) | Publication date |
|---|---|---|---|
| WO 2019201908 A1 | 24-10-2019 | NONE | |
| WO 2010003977 A1 | 14-01-2010 | AU 2009268025 A1 | 14-01-2010 |
| | | BR PI0915667 A2 | 17-01-2017 |
| | | CA 2729869 A1 | 14-01-2010 |
| | | CN 102143975 A | 03-08-2011 |
| | | CY 1119185 T1 | 14-02-2018 |
| | | CY 1124281 T1 | 22-07-2022 |
| | | DK 3211005 T3 | 07-06-2021 |
| | | EA 201100160 A1 | 30-08-2011 |
| | | EP 2315777 A1 | 04-05-2011 |
| | | EP 3211005 A1 | 30-08-2017 |
| | | ES 2633965 T3 | 26-09-2017 |
| | | ES 2875762 T3 | 11-11-2021 |
| | | HK 1158232 A1 | 13-07-2012 |
| | | HK 1243436 A1 | 13-07-2018 |
| | | HR P20171067 T1 | 06-10-2017 |
| | | HR P20210892 T1 | 26-11-2021 |
| | | HU E033803 T2 | 29-01-2018 |
| | | HU E054712 T2 | 28-09-2021 |
| | | IL 210204 A | 31-03-2015 |
| | | JP 6058264 B2 | 11-01-2017 |
| | | JP 6109869 B2 | 05-04-2017 |
| | | JP 6495361 B2 | 03-04-2019 |
| | | JP 2011527887 A | 10-11-2011 |
| | | JP 2015157812 A | 03-09-2015 |
| | | JP 2017158550 A | 14-09-2017 |
| | | KR 20110031969 A | 29-03-2011 |
| | | LT 2315777 T | 25-10-2017 |
| | | LT 3211005 T | 25-08-2021 |
| | | NZ 590515 A | 25-01-2013 |
| | | PL 2315777 T3 | 31-10-2017 |
| | | PL 3211005 T3 | 29-11-2021 |
| | | PT 2315777 T | 25-07-2017 |
| | | PT 3211005 T | 15-06-2021 |
| | | SI 2315777 T1 | 29-09-2017 |
| | | SI 3211005 T1 | 30-06-2021 |
| | | SM T201700366 T1 | 07-09-2017 |
| | | US 2011243962 A1 | 06-10-2011 |
| | | US 2014220026 A1 | 07-08-2014 |
| | | US 2017107274 A1 | 20-04-2017 |
| | | US 2018057569 A1 | 01-03-2018 |
| | | WO 2010003977 A1 | 14-01-2010 |
| | | ZA 201100446 B | 25-01-2012 |
| WO 2015181226 A1 | 03-12-2015 | AR 100642 A1 | 19-10-2016 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 24 30 7240

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2025

| Patent document cited in search report | Publication date | Patent family member(s) | Publication date |
|---|---|---|---|
| | | AU 2015265936 A1 | 15-12-2016 |
| | | BR 112016027671 A2 | 30-01-2018 |
| | | CA 2949884 A1 | 03-12-2015 |
| | | CN 106536550 A | 22-03-2017 |
| | | CN 114276444 A | 05-04-2022 |
| | | DK 3148582 T3 | 28-10-2019 |
| | | EA 201692471 A1 | 28-04-2017 |
| | | EC SP16091728 A | 30-06-2017 |
| | | EP 2949342 A1 | 02-12-2015 |
| | | EP 3148582 A1 | 05-04-2017 |
| | | ES 2752131 T3 | 03-04-2020 |
| | | HU E046187 T2 | 28-02-2020 |
| | | JP 2017519815 A | 20-07-2017 |
| | | KR 20170012376 A | 02-02-2017 |
| | | MX 374177 B | 05-03-2025 |
| | | PL 3148582 T3 | 15-06-2020 |
| | | PT 3148582 T | 04-11-2019 |
| | | RU 2689326 C1 | 27-05-2019 |
| | | SG 11201609886S A | 29-12-2016 |
| | | TW 201625679 A | 16-07-2016 |
| | | UA 120274 C2 | 11-11-2019 |
| | | US 2017101461 A1 | 13-04-2017 |
| | | US 2019263895 A1 | 29-08-2019 |
| | | WO 2015181226 A1 | 03-12-2015 |
| | | ZA 201608050 B | 27-05-2020 |
| US 2010304359 A1 | 02-12-2010 | CA 2684998 A1 | 29-01-2009 |
| | | EP 2145185 A2 | 20-01-2010 |
| | | JP 5214723 B2 | 19-06-2013 |
| | | JP 2010526297 A | 29-07-2010 |
| | | US 2010304359 A1 | 02-12-2010 |
| | | US 2016011183 A1 | 14-01-2016 |
| | | WO 2009014787 A2 | 29-01-2009 |
| EP 3351265 A1 | 25-07-2018 | AR 110949 A1 | 22-05-2019 |
| | | AU 2018210388 A1 | 01-08-2019 |
| | | BR 112019014773 A2 | 19-05-2020 |
| | | CA 3050286 A1 | 26-07-2018 |
| | | CN 110678196 A | 10-01-2020 |
| | | EA 201991736 A1 | 30-12-2019 |
| | | EP 3351265 A1 | 25-07-2018 |
| | | EP 3570878 A1 | 27-11-2019 |
| | | IL 267955 A | 26-09-2019 |
| | | JP 7139340 B2 | 20-09-2022 |
| | | JP 2020505383 A | 20-02-2020 |
| | | KR 20190119593 A | 22-10-2019 |

EPO FORM P0459

page 2 of 3

## ANNEX TO THE EUROPEAN SEARCH REPORT
## ON EUROPEAN PATENT APPLICATION NO.

EP 24 30 7240

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2025

| Patent document cited in search report | Publication date | Patent family member(s) | | Publication date |
|---|---|---|---|---|
| | | TW | 201831518 A | 01-09-2018 |
| | | UA | 126677 C2 | 11-01-2023 |
| | | US | 2019345232 A1 | 14-11-2019 |
| | | US | 2020308258 A1 | 01-10-2020 |
| | | WO | 2018136774 A1 | 26-07-2018 |
| | | WO | 2018136775 A1 | 26-07-2018 |

EPO FORM P0459

For more details about this annex : see Official Journal of the European Patent Office, No. 12/82

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019201908 A


**Non-patent literature cited in the description**

- **BELSHAW et al.** *PNAS*, 2004
- **GRÖGER et al.** *Int J Mol Sci*, 2020
- **BELSHAW R ; PEREIRA V ; KATZOURAKISA ; TALBOT G ; PACES J ; BURT A ; TRISTEM M.** Long-term reinfection of the human genome by endogenous retroviruses. *Proc Natl Acad Sci USA.*, 25 March 2004, vol. 101 (14), 4894-9
- **DEWANNIEUX et al.** *Genome Research*, 2006
- **GRÖGER V ; WIELAND L ; NAUMANN M ; MEINECKE AC ; MEINHARDT B ; ROSSNER S ; IHLING C ; EMMER A ; STAEGE MS ; CYNIS H.** Formation of HERV-K and HERV-Fc1 Envelope Family Members is Suppressed on Transcriptional and Translational Level. *Int J Mol Sci.*, 23 October 2020, vol. 21 (21), 7855
- **BEIMFORDE N ; HANKE K ; AMMAR I ; KURTH R ; BANNERT N.** Molecular cloning and functional characterization of the human endogenous retrovirus K113. *Virology*, 05 February 2008, vol. 371 (1), 216-25
- **FATIMA, S. ; SEN, P. ; SNEHA, P et al.** Hydrophobic Interaction Between Domain I of Albumin and B Chain of Detemir May Support Myristate-Dependent Detemir-Albumin Binding. *Appl Biochem Biotechnol*, 2017, vol. 182, 82-96

- **CHARVET B ; PIERQUIN J ; BRUNEL J ; GORTER R ; QUÉTARD C ; HORVAT B ; AMOR S ; PORTOUKALIAN J ; PERRON H.** Human Endogenous Retrovirus Type W Envelope from Multiple Sclerosis Demyelinating Lesions Shows Unique Solubility and Antigenic Characteristics. *Virol Sin.*, 26 March 2021, vol. 36 (5), 1006-1026
- **KOMURIAN-PRADEL F ; PARANHOS-BACCALA G ; BEDIN F ; OUNANIAN-PARAZ A ; SODOYER M ; OTT C ; RAJOHARISON A ; GARCIA E ; MALLET F ; MANDRAND B.** Molecular cloning and characterization of MSRV-related sequences associated with retrovirus-like particles. *Virology*, 20 July 1999, vol. 260 (1), 1-9
- **PERRON H ; JOUVIN-MARCHE E ; MICHEL M ; OUNANIAN-PARAZ A ; CAMELO S ; DUMON A ; JOLIVET-REYNAUD C ; MARCEL F ; SOUILLET Y ; BOREL E.** Multiple sclerosis retrovirus particles and recombinant envelope trigger an abnormal immune response in vitro, by inducing polyclonal Vbeta16 T-lymphocyte activation. *Virology*, 01 September 2001, vol. 287 (2), 321-32